Europäisches Patentamt

European Patent Office

Office européen des brevets

(19)

(11) **EP 0 814 753 B1**

(12) **EUROPÄISCHE PATENTSCHRIFT**

(45) Veröffentlichungstag und Bekanntmachung des Hinweises auf die Patenterteilung:
**13.11.2002   Patentblatt 2002/46**

(21) Anmeldenummer: **96907466.5**

(22) Anmeldetag: **14.03.1996**

(51) Int Cl.⁷: **A61K 7/00**, A61K 9/107

(86) Internationale Anmeldenummer:
**PCT/EP96/01088**

(87) Internationale Veröffentlichungsnummer:
**WO 96/028132 (19.09.1996 Gazette 1996/42)**

(54) **KOSMETISCHE ODER DERMATOLOGISCHE GELE AUF DER BASIS VON MIKROEMULSIONEN**

COSMETIC OR DERMATOLOGICAL GELS BASED ON MICROEMULSIONS

GELS COSMETIQUES OU DERMATOLOGIQUES A BASE DE MICROEMULSIONS

(84) Benannte Vertragsstaaten:
**AT BE CH DE ES FR GB IT LI NL SE**

(30) Priorität: **15.03.1995  DE 19509079**

(43) Veröffentlichungstag der Anmeldung:
**07.01.1998   Patentblatt 1998/02**

(73) Patentinhaber: **Beiersdorf Aktiengesellschaft**
**20245 Hamburg (DE)**

(72) Erfinder:
 • **DIEC, Khiet, Hien**
 **D-22523 Hamburg (DE)**
 • **GERS-BARLAG, Heinrich**
 **D-25495 Kummerfeld (DE)**
 • **KLIER, Manfred**
 **D-21521 Aumühle (DE)**
 • **SCHREIBER, Jörg**
 **D-22087 Hamburg (DE)**
 • **WOLF, Florian**
 **D-20251 Hamburg (DE)**

(74) Vertreter: **Dinné, Erlend**
**Beiersdorf AG**
**Unnastrasse 48**
**20245 Hamburg (DE)**

(56) Entgegenhaltungen:
**DE-A- 4 010 393          US-A- 4 052 331**

 • **GOLDSCHMIDT INFORMIERT, Bd. 57, 1982, BELGRADE (YUGOSLAVIA), Seiten 22-28, XP002013500 M. STUPAR: "Preparation of microemulsion gels with TEGO surfactants."**
 • **HAPPI HOUSHOLD & PERSONAL PRODUCTS INDUSTRY, Bd. 30, Nr. 2, 1993, RAMSEY, NJ, USA, Seiten 58-64, XP000336606 K. F. GALLAGHER: "Microemulsion Gels: A Formulator's Guide"**

**Beschreibung**

Die vorliegende Erfindung betrifft kosmetische oder dermatologische Gele auf der Basis von Mikroemulsionen, insbesondere solche Gele für kosmetische und dermatologische Zubereitungen. Als besondere Ausführungsform betrifft die vorliegende Erfindung kosmetische oder dermatologische Gele auf der Basis von Mikroemulsionen vom Typ Öl-in-Wasser, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 μm bis ca. 50 μm. Solche "Makroemulsionen" sind, ohne weitere färbende Zusätze, milchigweißgefärbt und opak. Feinere "Makroemulsionen", deren Tröpfchendurchmesser im Bereich von ca. $10^{-1}$ μm bis ca. 1 μm liegen, sind, wiederum ohne färbende Zusätze, bläulichweißgefärbt und undurchsichtig.

Mizellaren und molekularen Lösungen mit Partikeldurchmessern kleiner als ca. $10^{-2}$ μm, ist vorbehalten, klar und transparent zu erscheinen.

Der Tröpfchendurchmesser von transparenten bzw. transluzenten Mikroemulsionen dagegen liegt im Bereich von etwa $10^{-2}$ μm bis etwa $10^{-1}$ μm. Solche Mikroemulsionen sind meist niedrigviskos. Die Viskosität vieler Mikroemulsionen vom O/W-Typ ist vergleichbar mit der des Wassers.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten. Auch hier ist der hohe Gehalt an Emulgatoren von Nachteil.

Vorteil von Mikroemulsionsgelen ist, daß in der dispersen Phase Wirkstoffe feindispers vorliegen können. Ein weiterer Vorteil ist, daß sie aufgrund ihrer niedrigen Viskosität versprühbar sein können. Werden Mikroemulsionen als Kosmetika verwendet, zeichnen sich entsprechende Produkte durch hohe kosmetische Eleganz aus.

Es ist an sich bekannt, die Tröpfchen einer niedrigviskosen, insbesondere dünnflüssigen Mikroemulsion mit vernetzenden Substanzen miteinander zu verknüpfen, um auf diese Weise das dreidimensionale Netzwerk eines Geles zu erhalten.

In Nachr. Chem. Techn. Lab. 43 (1995) Nr. 1, S. 9 ff. werden zur Vernetzung von Mikroemulsionströpfchen kettenförmige, hydrophile Moleküle beschrieben, welche an den beiden Kettenenden je einen hydrophoben Rest aufweisen. Jene hydrophoben Reste tauchen in Mikroemulsionströpfchen ein, wobei die hydrophilen Kettenbereiche in der kontinuierlichen Wasserphase vorliegen. Im strengen Sinne ist es wohl nicht nötig, daß die hydrophoben Reste "eintauchen". Es kann im Einzelfalle auch durchaus genügen, wenn durch hydrophobe Wechselwirkung die hydrophoben Reste mit der Oberfläche der Mikroemulsionströpfchen in Kontakt treten und mehr oder weniger stark an dieser haften bleiben.

Als Vernetzer werden a.a.O. Polyoxyethylenglycole mit Oleylgruppen als hydrophobe Endgruppen angegeben.

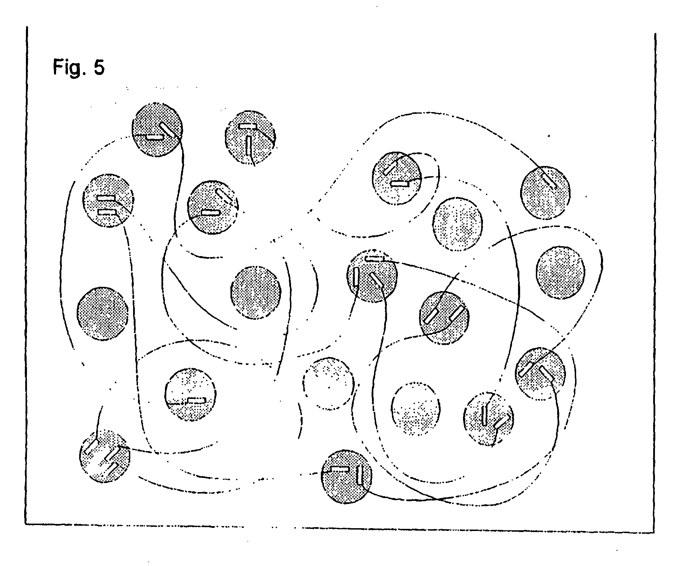

In Fig. 5 wird dieses Prinzip verdeutlicht: Die als schraffierte Kreise dargestellt Mikroemulsionströpfchen einer O/W-Mikroemulsion werden durch die als Linien dargestellten Vernetzermoleküle miteinander verbunden, welche an beiden Enden durch Rechtecke symbolisierte hydrophobe Reste tragen. Ersichtlich ist, daß ein Emulsionströpfchen grundsätzlich auch mehrere hydrophobe Reste beherbergen kann, wodurch eine stärkere Vernetzung und Dreidimensionalität des Netzwerks gewährleistbar ist.

Nachteilig an Mikroemulsionen, und somit auch an den Mikroemulsionsgelen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

An sich ist die Verwendung der üblichen kosmetischen Emulgatoren zwar unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mailorca-Akne" genannt. Eine Aufgabe der vorliegenden Erfindung war daher, Sonnenschutzprodukte zu entwickeln.

So betrifft die vorliegende Erfindung als besondere Ausführungsformen kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird im allgemeinen der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Mikroemulsionsgele eignen sich auch für andere kosmetische dermatologische Anwendungen, beispielsweise Desodorantien, so daß die vorliegende Erfindung in einer besonderen Ausführungsform Mikroemulsionsgele als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:

1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Auch die Verwendung von Mikroemulsionen als Grundlage für desodorierende oder antitranspirant wirkende Zubereitungen sind bekannt. Deren relativ hoher Gehalt an Emulgatoren, mit den geschilderten Nachteilen, war bisher ein Übelstand, dem es abzuhelfen galt.

Eine weitere Aufgabe der vorliegenden Erfindung war es also, Zubereitungen zu entwickeln, welche als Grundlage für kosmetische Desodorantien bzw. Antitranspirantien geeignet sind, und die Nachteile des Standes der Technik nicht aufweisen.

Weiterhin war es eine Aufgabe der Erfindung, kosmetische Grundlagen für kosmetische Desodorantien zu entwickeln, die sich durch gute Hautverträglichkeit auszeichnen.

Ferner war eine Aufgabe der vorliegenden Erfindung, Produkte auf der Basis von Mikroemulsionsgelen mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

In einer besonderen Ausführungsform betrifft die Erfindung daher Reinigungsemulsionen, insbesondere Gesichtsreinigungsemulsionen, bevorzugt Make-up-Entferner, beispielsweise Augenmake-up-Entferner.

Solche Zubereitungen sind an sich bekannt. Üblicherweise handelt es sich dabei um Abmischungen kosmetischer Öle oder wäßrige Zubereitungen oberflächenaktiver Substanzen, deren Funktion darin besteht, die Verunreinigung oder den Make-up-Körper zu solubilisieren und von der Haut zu entfernen.

Wasserfestes Augen-Make-up, beispielsweise Mascara, ist mit Make-up-Entfernern auf wäßriger Basis nur mit speziellen Tensiden zufriedenstellend zu entfernen. Diese Tenside besitzen aber oft eine nur begrenzte physiologische Verträglichkeit. Bei einem Kontakt solcher Stoffe mit der Schleimhaut, insbesondere der Augenschleimhaut, führen diese Stoffe zu Reizungen, die sich beispielsweise in einer Rötung der Augen äußern. Reaktionen dieser Art sind typisch für tensidhaltige Produkte.

Eine Aufgabe der vorliegenden Erfindung war mithin, solchen Problemen Abhilfe zu schaffen.

Die vorliegende Erfindung betrifft in einer weiteren Ausführungsform haarkosmetische Zubereitungen. Insbesondere betrifft die vorliegende Erfindung haarkosmetische Zubereitungen zur Pflege des Haars und der Kopfhaut. In einer bevorzugten Ausführungsform betrifft die vorliegende Erfindung Zubereitungen, die dazu dienen, das einzelne Haar zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt. femer dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet. welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stand der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Aufgabe war daher, auch diesen den Nachteilen des Standes der Technik Abhilfe zu schaffen.

Eine besondere Aufgabe der vorliegenden Erfindung war es, gelartige Zubereitungen auf Basis feindisperser Systeme vom Typ Öl-in-Wasser mit einem möglichst niedrigen Emulgatorgehalt zur Verfügung zu stellen, welche nicht die Nachteile des Standes der Technik aufweisen und welche für verschiedenste kosmetische und/oder dermatologische Anwendungen, beispielsweise die vorab beschriebenen Verwendungen finden können. Eine weitere Aufgabe der Erfindung war, das begrenzte Angebot an gelartigen Zubereitungen auf Basis feindisperser Systeme vom Typ Öl-in-Wasser des Standes der Technik zu bereichem.

Es ist an sich bekannt, daß hydrophile Emulgatoren, namentlich polyethoxylierte und polypropoxylierte Emulgatoren, bei steigender Temperatur ihr Löslichkeitsverhalten von wasserlöslich zu fettlöslich ändern. Ein Kennzeichen für die Hydrophilie eines gegebenen Emulgators ist dessen HLB-Wert.

Die Definition des HLB-Wertes ist für Polyolfettsäureester gegeben durch die Formel I

$$HLB = 20 * (1 - S/A)$$

Für eine Gruppe von Emulgatoren, deren hydrophiler Anteil nur aus Ethylenoxideinheiten besteht, gilt die Formel II

$$HLB = E/5$$

wobei

S = Verseifungszahl des Esters,
A = Säurezahl der zurückgewonnen Säure
E = Massenanteil Ethylenoxid (in %) am
Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Der Temperaturbereich, in dem die Emulgatoren ihre Löslichkeit ändern, wird Phaseninversionstemperaturbereich genannt. Für den Phaseninversionstemperaturbereich soll innerhalb dieser Schrift auch die Abkürzung "PIT" gebraucht werden.

Die Änderung dieses Löslichkeitsverhaltens äußert sich bekanntermaßen darin, daß eine Mischung aus Wasser, Öl und O/W-Emulgatoren, welche unterhalb des PIT nach Rühren eine O/W-Emulsion ergibt, auf eine Temperatur oberhalb des PIT gebracht wird, typischerweise etwa 70-90° C, als Zwischenstufe den Zustand einer Mikroemulsion durchlaufen kann, um schließlich oberhalb des PIT eine W/O-Emulsion zu ergeben. Wird diese Emulsion abgekühlt, wird wieder eine O/W-Emulsion erhalten, welche aber eine Tröpfchengröße von bis zu 200 nm besitzt und dabei im Bereich zwischen einer Mikroemulsion und einer feinen Makroemulsion liegt.

Auf solche Weise hergestellte Mikroemulsionen des Standes der Technik haben allerdings den Nachteil, daß erstens die Tröpfchengröße immer noch recht hoch ist, daß die Emulsion bei Raumtemperatur opak weiß bis bläulich ist und/oder immer noch ein hoher Anteil an einem oder mehreren Emulgatoren nötig ist.

Weiterhin ist nachteilig, daß auf solche Weise hergestellte Mikroemulsionen zwar bei hoher Temperatur, also beispielsweise im PIT, praktisch transparent sind, aber beim Absinken auf Raumtemperatur wieder undurchsichtig werden. Auf solchen Mikroemulsionen beruhende Gele sind also bestenfalls unschön, haben auch in technischer, galenischer und kosmetischer Sicht Nachteile.

Auch diesen Übelständen galt es also, abzuhelfen.

Erstaunlicherweise werden alle der Erfindung zugrundliegenden Aufgaben gelöst durch wie folgt:

(a) beruhend auf Mikroemulsionen vom Typ Öl-in-Wasser, welche umfassen

- eine diskontiniuerliche Ölphase und eine kontinuierliche Wasserphase
- enthaltend mindestens einen polyethoxylierten bzw. polypropoxylierten bzw. polyethoxylierten und polypropoxylierten O/W-Emulgator
- wobei der polyethooxylierte bzw. polypropoxylierte bzw. polyethoxylierte und polypropoxylierte O/W-Emulgator oder die polyethooxylierten bzw. polypropoxylierten bzw. polyethoxylierten und polypropoxylierten O/W-Emulgatoren gewählt wird oder werden aus der Gruppe
- der Fettalkoholethoxylate der allgemeinen Formel $R-O-(-CH_2-CH_2-O-)_n-H$, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel $R-O-(-CH_2-CH_2-O-)_n-R'$, wobei R und R' unabhängig voneinander verzweigte oder unverzweigte Alkyl- oder Alkenylreste und n eine Zahl von 10 bis 80 darstellen
- der Fettsäureethoxylate der allgemeinen Formel $R-COO-(-CH_2-CH_2-O-)_n-H$, wobei R einen verzweigten oder unverzweigten Alkyl- oder Alkenylrest und n eine Zahl von 10 bis 40 darstellen,
- der veretherten Fettsäureethoxylate der allgemeinen Formel $R-COO-(-CH_2-CH_2-O-)_n-R'$, wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel $R-COO-(-CH_2-CH_2-O-)_n-C(O)-R'$, wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweiger Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel $R-O-(-CH_2-CH_2-O-)_n-CH_2-COOH$ bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel $R-O-(-CH_2-CH_2-O-)_n-SO_3-H$ mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.

- der Fettalkoholpropoxylate der allgemeinen Formel R-O-(-CH$_2$-CH(CH$_3$)-O-)$_n$-H, wobei R einen verzweigten oder unverzweigten Alkyl- oder Alkenylrest und n eine Zahl von 10 bis 80 darstellen,
- der Polypropylenglycolether der allgemeinen Formel R-O-(-CH$_2$-CH(CH$_3$)-O-)$_n$-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-CH$_2$-CH(CH$_3$)-O-)$_n$-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-CH$_2$-CH(CH$_3$)-O-)$_n$-C(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel R-COO-(-CH$_2$-CH(CH$_3$)-O-)$_n$-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel R-O-(-CH$_2$-CH(CH$_3$)O-)$_n$-CH$_2$-COOH bzw. deren kosmetisch oder pharmazeutisch akzeptablen Salze, wobei R einen verzweigten oder unverzweigten Alkyloder Alkenylrest und n eine Zahl von 3 bis 50 darstellen,
- der Alkylethersulfate bzw. die diesen Sulfaten zugrundeliegenden Säuren der allgemeinen Formel R-O-(-CH$_2$-CH(CH$_3$)-O-)$_n$-SO$_3$-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel R-O-X$_n$-Y$_m$-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel R-O-X$_n$-Y$_m$-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel R-COO-X$_n$-Y$_m$-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel R-COO-X$_n$-Y$_m$-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.
- gewünschtenfalls enthaltend einen oder mehrere W/O-Emulgatoren
- einen Emulgatorgehalt kleiner als 20 Gew.-%, bezogen auf das Gesamtgewicht der Emulsion, aufweisend,
- erhältlich auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusätz- und/oder Wirkstoffe auf eine Temperatur innerhalb oder oberhalb des Phaseninversionstemperaturbereiches bringt, und hernach auf Raumtemperatur abkühlt,

(b) bei welcher die Tröpfchen der diskontinuierlichen Ölphase durch eine oder mehrere Vernetzersubstanzen miteinander verbunden sind, gewählt aus der Gruppe

- der Polyethylenglycolether der allgemeinen Formel R-O-(-CH$_2$-CH$_2$-O-)$_n$-R', wobei R und R' unabhängig voneinander verzweigte oder unverzweigte Alkyl-, Aryl- oder Alkenylreste und n eine Zahl größer als 100 darstellen,
- der veretherten Fettsäureethoxylate der allgemeinen Formel R-COO-(-CH$_2$-CH$_2$-O-)$_n$-R', wobei R und R' unabhängig voneinander verzweigte oder unverzweigte Alkyl-, Aryl- oder Alkenylreste und n eine Zahl größer als 100 darstellen,

- der veresterten Fettsäureethoxylate der allgemeinen Formel R-COO-(-CH$_2$-CH$_2$-O-)$_n$-C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-(-CH$_2$-CH(CH$_3$)-O-)$_n$-R', wobei R und R' unabhängig voneinander verzweigte oder unverzweigte Alkyl-, Aryl- oder Alkenylreste und n eine Zahl größer als 100 darstellen, der veresterten Fettsäurepropoxylate der allgemeinen Formel R-COO-(-CH$_2$-CH(CH$_3$)-O-)$_n$-C(O)-R', wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel R-O-X$_n$-Y$_m$-R', wobei R und R' unabhängig voneinander verzweigte oder unverzweigte Alkyl-, Äryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist

- der veretherten Fettsäurepropoxylate der allgemeinen Formel R-COO-X$_n$-Y$_m$-R', wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist.

Es ist dabei gleichermaßen vorteilhaft, wenn die Vernetzersubstanz, im Rahmen der vorliegenden Beschreibung auch als Verdicker bezeichnet, ein eigenständiges Gelnetzwerk bildet, in welchem die Mikroemulsionströpfchen dann durch hydrophobe Wechselwirkung festgehalten werden (dann liegen sogenannte assoziative Verdicker vor), oder ob der Zusammenhalt des Netzes durch die Vernetzung mit den Mikroemulsionströpfchen in den Knotenpunkten des Netzes bewirkt wird.

Zwar beschreiben die DE 40 10 393 und die US - 4,052,331 Systeme auf der Grundlage von Mikroemulsionen. Sie konnten aber nicht den Weg zur vorliegenden Erfindung weisen.

Insbesondere vorteilhaft sind das PEG-150-Distearat und das PEG-150-Dioleat. Auch das PEG-300-Pentaerythrityltetraisostearat, das PEG-120 Methylglucosedioleat, das PEG-160-Sorbitantriisostearat, das PEG-450-Sorbitolhexaisostearat und das PEG-230-Glyceryltriisostearat sind vorteilhaft als Verdicker zu verwenden.

Die erfindungsgemäßen Zubereitungen können vorteilhaft 0,001 - 20 Gew.-% eines oder mehrerer erfindungsgemäß verwendeter Verdicker enthalten. Bevorzugt ist im allgemeinen, den Gehalt an Verdickern kleiner als 10 Gew.-% zu wählen, insbesondere kleiner als 5 Gew.-%.

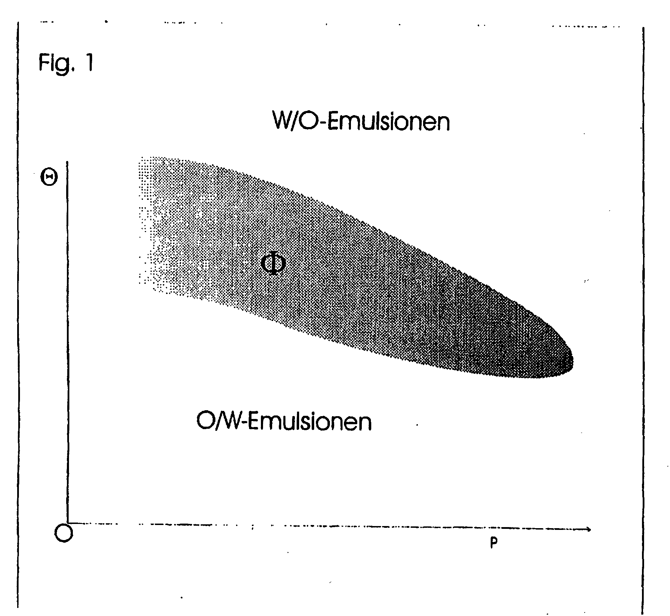

In Fig. 1 wird eine stark vereinfachte Darstellung eines Phasendiagrammes wiedergegeben. Der variable Parameter P wird gegen die Temperatur $\theta$ als zweite Variable aufgetragen. P stellt dabei einen Konzentrationsparameter dar, entweder den Anteil der Ölphase, den Anteil der Wasserphase oder die Konzentration eines Emulgators oder eines Emulgatorgemisches. Für erfindungsgemäße Systeme gilt dabei, daß bei niedrigeren Temperaturen eine O/W-Emulsion vorliegt und bei Erhöhung der Temperatur der Phaseninversionsbereich durchlaufen werden kann. Bei weiterer Erhöhung der Temperatur werden W/O-Emulsionen beobachtet. Die Struktur des Systems im Phaseninversionsbereich ist dem Anschein nach nicht kritisch für die vorliegende Erfindung. Denkbar ist beispielsweise, daß im Phaseninversionsbereich lamellare Phasen, bikontinuierliche Phasen, kubische, hexagonale bzw. invers hexagonale Phasen vorliegen, auch daß der Phaseninversionsbereich aus mehreren gleichartigen oder mehr oder weniger unterschiedlichen Phasen zusammengesetzt ist.

Der Phaseninversionsbereich läßt sich mathematisch darstellen als Punktmenge innerhalb des geradlinigen Koordinatensystems $\Sigma$, welches durch die Größen Temperatur, der Konzentration eines geeigneten Emulgators bzw. eines Emulgatorengemisches in der Zubereitung sowie die jeweiligen Konzentrationen der Ölphase und der Wasserphase gebildet wird, gemäß:

$$\Sigma = \{O, \theta, m, H, W\},$$

mit

O - Koordinatenursprung
$\theta$ - Temperatur
m - Konzentration des Emulgators/Emulgatorengemisches
H - Konzentration der Ölphase
W - Konzentration der Wasserphase

Dabei muß genaugenommen natürlich in einem mehrkomponentigen Emulgatorensystem der Beitrag $m_i$ je-

des einzelnen Emulgators zur Gesamtfunktion berücksichtigt werden, was bei einem i-komponentigen Emulgatorensystem zur Beziehung

$$\Sigma = \{O, \theta, m_1, m_2, ..., m_i, H, W\}$$

führt.

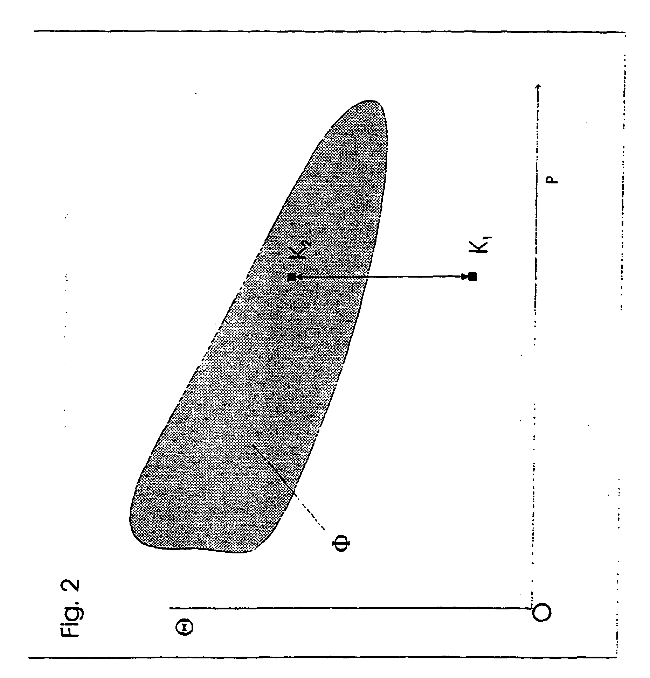

Der Phaseninversionsbereich $\Phi$ stellt dabei im mathematischen Sinne ein zusammenhängendes Gebiet oder eine Vielzahl zusammenhängender Gebiete innerhalb des Koordinatensystems $\Sigma$ dar. $\Phi$ repräsentiert die Gesamtmenge der Koordinatenpunkte $K(\theta, a, m_1, m_2, ..., m_1, H, W)$, welche erfindungsgemäße Gemische aus Wasserphase der Konzentration W, Ölphase der Konzentration H, i erfindungsgemäßen Emulgatoren der Konzentration $m_i$ bei der Temperatur $\theta$ bestimmen, und für welche gilt, daß beim Übergang von einer Koordinate $K_1 \notin \Phi$ zu einer Koordinate $K_2 \in \Phi$ Phaseninversion eintritt, wie in Fig. 2 beschrieben.

Unerheblich ist dabei, ob der Phaseninversionsbereich eines gegebenen Systems ein einziges zusammenhängendes (i + 3)-dimensionalen Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbarung wird daher stets verallgemeinernd von "dem" oder "einem" Phaseninversionsbereich gesprochen, auch bei Vorliegen zweier oder mehrerer voneinander getrennter solcher Bereiche.

Als variable Koordinaten in Fig.2 werden Temperatur $\theta$ und die der vorgeschilderte Konzentrationsparameter P angegeben, wobei offengelassen bleiben kann, um welchen speziellen Konzentrationsparameter es sich handelt. Beim Übergang von $K_1$ nach $K_2$ wird lediglich die Temperatur erhöht, die anderen Variablen werden konstant gehalten.

Unter den erfindungsgemäßen Bedingungen ist dieser Prozeß nicht reversibel, d.h., kehrt das System von der Koordinate $K_2 \in \Phi$ wieder zur Kcordinate $K_1 \notin \Phi$ zurück, können, transparente O/W-Mikroemulsionen erhalten werden, welche bei Anwesenheit oder unter Zugabe erfindungsgemäß verwendeter Verdicker, erfindungsgemäße Mikroemulsionsgele zugänglich machen.

Die Praxis der Herstellung eines erfindungsgemäßen Mikroemulsionsgeles besteht demgemäß vorteilhaft darin, nach Auswahl geeigneter Rohstoffe, d.h., Wasser- und Ölphase, ein oder mehrere erfindungsgemäß veiwendete O/W-Emulgatoren, letzterer oder letztere vorliegend in Konzentrationen, bei welchen Phaseninversion für das gegebene Gemisch möglich ist, ein oder mehrere erfindungsgemäß verwendete Verdicker und gegebenenfalls weitere Substanzen, die Einzelkomponenten unter Rühren zusammenzugeben, durch Erhöhung der Temperatur des Gemisches Phaseninversion herbeizuführen, hernach unter fortwährendem Rühren das Gemisch auf Raumtemperatur abkühlen zu lassen, wobei der oder die erfindungsgemäß verwendeten Verdicker zu jedem Zeitpunkte der Herstellung beigefügt werden können.

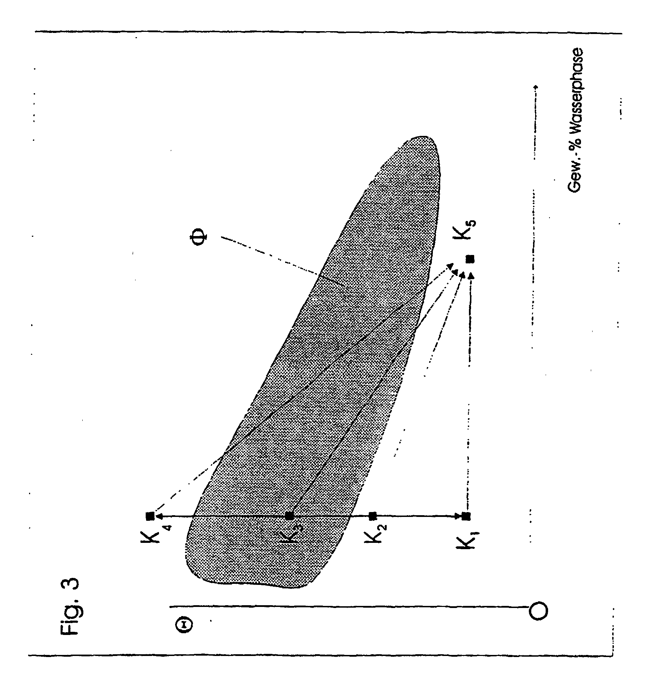

Es ist dabei aber auch möglich, mehrere Parameter zugleich zu variieren, wie in Fig.3 angezeigt. In Fig. 3 wird die Konzentration der Wasserphase gegen die Temperatur aufgetragen. Ausgehend von der Koordinate $K_1 \notin \Phi$ können durch Erhöhung der Temperatur, unter Beibehaltung aller anderen Parameter, die Koordinaten $K_2 \notin \Phi$ und $K_4 \notin \Phi$ erreicht werden bzw. $K_3 \in \Phi$. Ausgehend von den Koordinaten $K_3$ und $K_4$ können durch Senken der Temperatur, unter Beibehaltung aller anderen Parameter, zurück zur Koordinate $K_1$ O/W-Mikroemulsionen erhalten werden, welche bei Anwesenheit oder unter Zugabe erfindungsgemäß verwendeter Verdicker, erfindungsgemäße Mikroemulsionsgele zugänglich machen.

Ausgehend von den Koordinaten $K_3$ und $K_4$ kann durch Senken der Temperatur. und durch zusätzliche Variation der Konzentration der Ölphase, in Fig.3 durch Zugabe von Wasser. die Koordinate $K_5$ erreicht und können O/W-Mikroemulsionen erhalten werden, welche bei Anwesenheit oder unter Zugabe erfindungsgemäß verwendeter Verdicker, erfindungsgemäße Mikroemulsionsgele zugänglich machen.

Es ist angesichts der Fig. 3 konsequent, daß, ausgehend von der Koordinate $K_4$, obwohl dieser außerhalb des Phaseninversionsbereiches befindlich ist, Systeme erhalten werden können, ähnlich denen, die von $K_3$ ausgehen, da ja auch ausgehend von $K_4$ bei Senkung der Temperatur der Phaseninversionsbereich zwangsläufig durchschritten werden muß.

Auch ausgehend von der Koordinate $K_1$ kann durch Variation der Konzentration der Wasserphase, also beispielsweise durch Zugabe von Wasser, wie in Fig. 3 aufgeführt, die Koordinate $K_5$ erreicht und können O/W-Mikroemulsionen erhalten werden, welche bei Anwesenheit oder unter Zugabe erfindungsgemäß verwendeter Verdicker, erfindungsgemäße Mikroemulsionsgele zugänglich machen. Dazu muß allerdings vorausgeschickt werden, daß in diesem Falle bereits eine O/W-Mikroemulsion, gewissermaßen als Konzentrat, vorliegen muß, welches dann durch Verdünnen zu einer O/W-Mikroemulsion veränderter Zusammensetzung umgesetzt wird, welche bei Anwesenheit oder unter Zugabe erfindungsgemäß verwendeter Verdicker, erfindungsgemäße Mikroemulsionsgele zugänglich macht.

Es war jedoch nach allem erstaunlich und verfügt daher über eigenständige erfinderische Tätigkeit, daß auch ausgehend von der Koordinate $K_2$, welche außerhalb des Phaseninversonsbereiches liegt, sei es bei einfacher Variation der Temperatur zurück zur Koordinate $K_1$ oder zusätzlicher Variation der Konzentration der Ölphase, also beispielsweise durch zusätzliches Verdünnen mit einer Wasserphase zur Koordinate $K_5$, O/W-Mikroemulsionen erhältlich sind, welche bei Anwesenheit oder unter Zugabe erfindungsgemäß verwendeter Verdicker, erfindungsgemäße Mikroemulsionsgele zugänglich machen, ohne daß Phaseninversion durchlaufen wird.

Dies geschieht vorteilhaft auf die Weise, daß ein Gemisch aus den Grundkomponenten, umfassend Wasserphase, Ölphase, einen oder mehrere der erfindungsgemäß verwendeten O/W-Emulgator gewünschtenfalls einen oder mehrere W/O-Emulgatoren, einen oder mehrere erfindungsgemäß verwendete Verdicker sowie gewünschtenfalls weitere Hilfs-, Zusatz- und/oder Wirkstoffe, welche unterhalb des Phaseninversionstemperaturbereiches eine O/W-Emulsion bilden, auf eine Temperatur bringt,

- bei welcher die in der Ölphase löslichen Komponenten entweder gelöst oder zumindest in geschmolzenem Zustande vorliegen
- und welche mindestens der Schmelztemperatur der höchstschmelzenden, nicht in gelöstem Zustande vorliegenden Ölkomponente entspricht,
- welche unterhalb des Phaseninversionstemperaturbereiches des Systemes liegt,
- das entstandene Gemisch hemach auf Raumtemperatur abkühlt
- wobei der oder die erfindungsgemäß verwendeten Verdicker zu jedem Zeitpunkte der Herstellung beigefügt werden können.

Dieses erfindungsgemäße Verfahren ist besonders gut geeignet, wenn den erfindungsgemäßen O/W-Mikroemulsionsgelen wärmeempfindliche oder leichtflüchtige Substanzen einverleibt werden sollen. Darüberhinaus ist dieses bei relativ niedrigen Temperaturen durchzuführende Verfahren gegenüber üblichen Verfahren energiesparend.

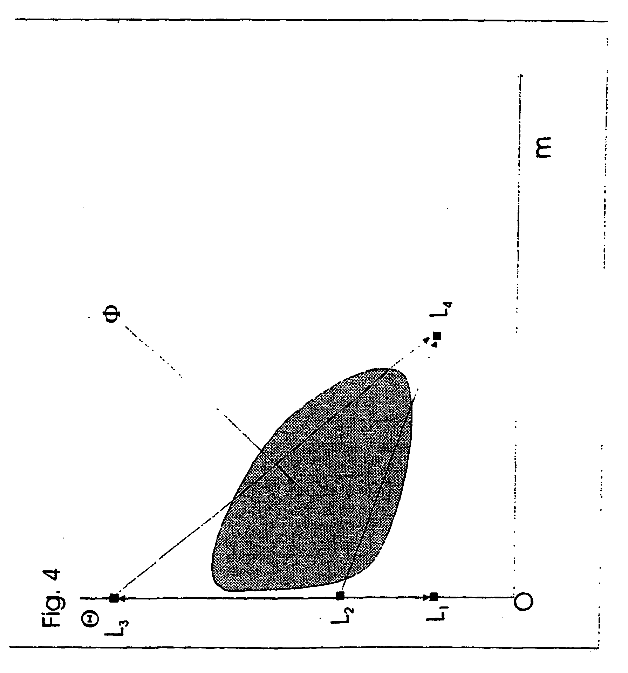

In Fig. 4 wird der Fall beschrieben, in welchem in der Koordinate $L_1$ zunächst kein erfindungsgemäßer O/W-Emulgator vorliegt, und in welchem das System durch Erhöhen der Temperatur auf eine Koordinaten $L_3 \notin \Phi$ oder auf eine Koordinate $L_2 \notin \Phi$ gebracht wird. Selbstverständlich kann die Koordinate $L_2$ auch durch Abkühlen eines in der Koordinate $L_3$ vorliegenden Systems erreicht werden. Die Koordinaten $L_2$ und $L_3$, in denen beispielsweise W/O-Emulsionen vorliegen können, unterscheiden sich prinzipiell lediglich dadurch, daß die $L_3$ zugeordnete Temperatur höher ist als jede Temperatur, die dem Phaseninversionstemperaturbereiche zugeordnet werden kann.

Die Gegenwart eines zusätzlichen W/O-Emulgators für Systeme, die in Fig. 4 symbolisiert werden, ist nicht unbedingt erforderlich, allerdings vorteilhaft. Zugabe eines erfindungsgemäßen O/W-Emulgators oder mehrerer solcher Emulgatoren in den Koordinaten $L_2$ oder $L_3$, bei Senkung der Temperatur, befördert das System zur Koordinate $L_4$, bei welcher dann eine O/W-Mikroemulsion vorliegt, welche bei Anwesenheit oder unter Zugabe erfindungsgemäß verwendeter Verdicker, erfindungsgemäße Mikroemulsionsgele zugänglich machen.

Eine weitere vorteilhafte Ausführungsform des erfindungsgemäßen Verfahrens besteht demgemäß darin, nach der Auswahl geeigneter Rohstoffe, d.h., Wasser- und Ölphase und gegebenenfalls weitere Substanzen, die Einzelkomponenten unter Rühren auf eine Temperatur zu bringen, bei welcher Phaseninversion für das gegebene Gemisch möglich ist. und durch Zugabe des erfindungsgemäß verwendeten O/W-Emulgators oder der erfindungsgemäß verwendeten O/W-Emulgatoren zum Gemisch Phaseninversion herbeizuführen, hemach unter fortwährendem Rühren das Gemisch auf Raumtemperatur abkühlen zu lassen, wobei der oder die erfindungsgemäß verwendeten Verdicker zu jedem Zeitpunkte der Herstellung beigefügt werden können.

Es übersteigt nicht das Können des Fachmannes, durch einfache Versuche den geeigneten Temperaturbereich zu ermitteln, innerhalb dessen ein gegebenes Gemisch Phaseninversion durchlaufen kann. Überlicherweise ist dieser Temperaturbereich zwischen 70 und 95° C zu wählen, kann im Einzelfalle auch darüber oder darunter angesiedelt sein.

In der Praxis ist möglich und gegebenenfalls sogar vorteilhaft, bei der Herstellung einer erfindungsgemäßen Mikroemulsion den Temperaturbereich, der dem Phaseninversionsbereich zugeordnet werden kann, auch zu übersteigen, da beim Abkühlen auf Raumtemperatur dieser Bereich dann zwangsläufig durchlaufen wird.

Insbesondere ist vorteilhaft, wenn der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt wird oder werden aus der Gruppe

- der Fettalkoholethoxylate der allgemeinen Formel R-O-(-CH$_2$-CH$_2$-O-)$_n$-H, wobei R einen verzweigten oder unverzweigten Alkyl- oder Alkenylrest mit 5 - 30 C-Atomen und n eine Zahl von 10 bis 25 darstellen
- der ethoxylierten Wollwachsalkohole mit HLB-Werten von 11 - 16, ganz besonders vorteilhaft mit mit HLB-Werten von 14,5 -15,5.
- der Polyethylenglycolether der allgemeinen Formel R-O-(-CH$_2$-CH$_2$-O-)$_n$-R', wobei R und R' unabhängig voneinander verzweigte oder unverzweigte Alkyl- oder Alkenylreste mit 5 - 30 C-Atomen und n eine Zahl von 10 bis 25 darstellen,

- der Fettsäureethoxylate der allgemeinen Formel R-COO-(-CH$_2$-CH$_2$-O-)$_n$-H, wobei R einen verzweigten oder unverzweigten Alkyl- oder Alkenylrest mit 5 - 30 C-Atomen und n eine Zahl von 10 bis 25 darstellen,
- der veretherten Fettsäureethoxylate der allgemeinen Formel R-COO-(-CH$_2$-CH$_2$-O-)$_n$-R', wobei R und R' unabhängig voneinander verzweigte oder unverzweigte Alkyl- oder Alkenylreste mit 5 - 30 C-Atomen und n eine Zahl von 10 bis 50 darstellen,
- der veresterten Fettsäureethoxylate der allgemeinen Formel R-COO-(-CH$_2$-CH$_2$-O-)$_n$-C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polyethylenglycolglycerinfettsäureester gesättigter und/oder ungesättigter, verzweigter und/oder unverzweiger Fettsäuren mit 6 bis 26 C-Atomen und einem Ethoxylierungsgrad zwischen 3 und 40
- der ethoxylierten Sorbitanester mit einem Ethoxylierungsgrad von 3 bis 30
- der Cholesterinethoxylate mit HLB-Werten von 11 - 16, ganz besonders vorteilhaft mit HLB-Werten von 14,5 - 15,5
- der ethoxylierten Triglyceride mit HLB-Werten von 11 - 16, ganz besonders vorteilhaft mit mit HLB-Werten von 14,5 - 15,5
- der Alkylethercarbonsäuren der allgemeinen Formel R-O-(-CH$_2$-CH$_2$-O-)$_n$-CH$_2$-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 20 darstellen,
- der Polyoxyethylensorbitolfettsäureester, basierend auf verzweigten oder unverzweigten Alkan- oder Alkensäuren und einen Ethoxylierungsgrad von 10 bis 80 aufweisend, beispielsweise vom Sorbeth-Typ,
- der Alkylethersulfate bzw. die diesen Sulfaten zugrundeliegenden Säuren der allgemeinen Formel R-O-(-CH$_2$-CH$_2$-O-)$_n$-SO$_3$-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 3 bis 30 darstellen.
- der Fettalkoholpropoxylate der allgemeinen Formel R-O-(-CH$_2$-CH(CH$_3$)-O-)$_n$-H, wobei R einen verzweigten oder unverzweigten Alkyloder Alkenylrest mit 5 - 30 C-Atomen und n eine Zahl von 10 bis 30 darstellen,
- der Polypropylenglycolether der allgemeinen Formel R-O-(-CH$_2$-CH(CH$_3$)-O-)$_n$-R', wobei R und R' unabhängig voneinander verzweigte oder unverzweigte Alkyl- oder Alkenylreste mit 5 - 30 C-Atomen und n eine Zahl von 10 bis 40 darstellen,
- der propoxylierten Wollwachsalkohole mit HLB-Werten von 11 - 16, ganz besonders vorteilhaft mit HLB-Werten von 14,5 - 15,5,
- der Fettsäurepropoxylate der allgemeinen Formel R-COO-(-CH$_2$-CH(CH$_3$)-O-)$_n$-H, wobei R einen verzweigten oder unverzweigten Alkyloder Alkenylrest mit 5 - 30 C-Atomen und n eine Zahl von 10 bis 40 darstellen.
- der veretherten Fettsäurepropoxylate der allgemeinen Formel R-COO-(-CH$_2$-CH(CH$_3$)-O-)$_n$-R', wobei R und R' unabhängig voneinander verzweigte oder unverzweigte Alkyl- oder Alkenylreste mit 5 - 30 C-Atomen und n eine Zahl von 10 bis 30 darstellen,
- der veresterten Fettsäurepropoxylate der allgemeinen Formel R-COO-(-CH$_2$-CH(CH$_3$)-O-)$_n$-C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polypropylenglycolglycerinfettsäureester gesättigter und/oder ungesättigter, verzweigter und/oder unverzweiger Fettsäuren mit 6 bis 26 C-Atomen und einem Propoxylierungsgrad zwischen 3 und 50
- der propoxylierten Sorbitanester mit einem Propoxylierungsgrad von 3 bis 80
- der Cholesterinpropoxylate mit HLB-Werten von 11 - 16, ganz besonders vorteilhaft mit mit HLB-Werten von 14,5 - 15,5
- der propoxylierten Triglyceride mit HLB-Werten von 11 - 16, ganz besonders vorteilhaft mit mit HLB-Werten von 14,5 - 15,5
- der Alkylethercarbonsäuren der allgemeinen Formel R-O-(-CH$_2$-CH(CH$_3$)O-)$_n$-CH$_2$-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 30 darstellen,
- der Alkylethersulfate bzw. die diesen Sulfaten zugrundeliegenden Säuren der allgemeinen Formel R-O-(-CH$_2$-CH(CH$_3$)-O-)$_n$-SO$_3$-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 30 darstellen.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 16, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19). Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12). Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist femer von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat. Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat.

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.
Als Alkylethersulfat kann Natrium Laureth 1-4 sulfat vorteilhaft verwendet werden.
Als ethoxyliertes Cholesterinderivat kann vorteilhaft Polyethylenglycol(30)Cholesterylether verwendet werden. Auch Polyethylenglycol(25)Sojasterol hat sich bewährt.
Als ethoxylierte Triglyceride können vorteilhaft die Polyethylenglycol(60) Evening Primrose Glycerides verwendet werden (Evening Primrose = Nachtkerze)
Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat. Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.
Es ist ebenfalls günstig, die Sorbitanester aus der Gruppe Polyethylenglycol(20)sorbitanmonolaurat, Polyethylenglycol(20)sorbitanmonostearat. Polyethylenglycol(20)sorbitanmonoisostearat, Polyethylenglycol(20)sorbitan-monopalmitat. Polyethylenglycol(20)-sorbitanmonoooleat zu wählen.

Als fakultative, dennoch erfindungsgemäß vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24. insbesondere 12 - 18 C-Atomen, Monoglycerinether gesättigter und/oder ungesättigter, verzweigter und/oder unverzweigter Alkohole einer Kettenlänge von 8 bis 24. insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweiger Alkan-carbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmo-nomyristat, Glycerytmonoooleat, Diglycerylmonostearat, Diglycerylmonoisostearat, Propylenglycolmonostearat, Propy-lenglycolmonoisostearat, Propylenglycolmonocaprylat, Propylenglycolmonolaurat, Sorbitanmonoisostearat, Sorbitan-monolaurat, Sorbitanmonocaprylat, Sorbitanmonoisoooleat, Saccharosedistearat, Cetylalkohol, Stearylalkohol, Arachi-dylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es ist erfindungsgemäß möglich, den Gesamtgehalt an Emulgatoren kleiner als 15 Gew.-%, bezogen auf das Gesamtgewicht der erfindungsgemäßen Mikroemulsionsgele, zu halten. Es wird bevorzugt, den gesamtgehalt an Emulgatoren kleiner als 10 Gew.%, insbesondere kleiner als 8 Gew.-%, bezogen auf das Gesamtgewicht der Mikroemulsionsgele, zu halten.

Die Ölphase der erfindungsgemäßen Mikroemulsionsgele wird vorteilhaft gewählt aus der Gruppe der Ester aus gesättigten und/oder, ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Auch beliebige Abmischungen solcher Öl- und Wachskomponenten sind vorteilhaft im Sinne der vorliegenden Erfindung einzusetzen.

Es kann auch gegebenenfalls vorteilhaft sein, Wachse, beispielsweise Cetylpalmitat, als alleinige Lipidkomponente der Ölphase einzusetzen. In solchen Fällen können die erfindungsgemäßen O/W-Mikroemulsionen auch gegebenenfalls als Mikrodispersionen fester Wachspartikel anfallen.

Vorteilhaft wird die Ölphase gewählt aus der Gruppe 2-Ethylhexylisostearat, Octyldodecanol, Isotridecyliso-nonanoat, Isoeicosan, 2-Ethylhexylcocoat, $C_{12-15}$-Alkylberzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase femer einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Als vorteilhafte Verkörperung der vorliegenden Erfindung wird ebenfalls angesehen ein Verfahren zur Herstellung von O/W-Mikroemulsionsgelen, welche umfassen:

(1) eine Wasserphase, gewünschtenfalls umfassend übliche, in Wasser lösliche oder dispergierbare Substanzen,
(2) eine Ölphase, welche im wesentlichen aus schwerflüchtigen Bestandteilen zusammengesetzt ist und welche gewünschtenfalls übliche, in der Ölphase lösliche oder dispergierbare Substanzen umfaßt,
(3) einen oder mehrere polyethoxylierte O/W-Emulgator und/oder
einen oder mehrere polypropoxylierte O/W-Emulgatoren und/oder
einen oder mehrere polyethoxylierte und polypropoxylierte O/W-Emulgatoren,
(4) gewünschtenfalls einen oder mehrere W/O-Emulgatoren,
(5) einen erfindungsgemäßen Verdicker,

dadurch gekennzeichnet, daß

(a) die Anfangskonzentrationen der Ölphase, der Wasserphase und gewünschtenfalls eines oder mehrerer W/O-Emulgatoren gewählt werden und diese Bestandteile zueinander gegeben werden,
(b) die Anfangskonzentration des oder der O/W-Emulgatoren, welche auch gegebenenfalls gleich Null sein kann, gewählt wird und dieser oder diese O/W-Emulgatoren zu dem in (a) erhaltenden Gemisch gegeben werden
(c) wobei das in (b) erhaltene Gemisch eine Ausgangstemperatur besitzt
(d) das in (b) erhaltende Gemisch durch geeignete Variation mindestens eines Parameters, gewählt aus der Gruppe Temperatur und der Konzentration bzw. Konzentrationen mindestens eines der gewählten Emulgatoren und/oder der Ölphase und/oder der Wasserphase,das so gebildete Gemisch den Phaseninversionsbereich zwischen W/O-Emulsionen und O/W-Emulsionen durchläuft und in den Bereich gebracht wird, wo das Gemisch als O/W-Emulsion bzw. O/W-Mikroemulsion vorliegt,
(e) das in (d) erhaltene Gemisch sodann gegebenenfalls weiteren Aufbereitungsschritten unterworfen wird.
(f) wobei der oder die erfindungsgemäß verwendetenen Verdicker zu jedem Zeitpunkte der Herstellung beigefügt werden können.

Erfindungsgemäß gleichermaßen vorteilhaft sind Verfahren, bei welchen die Variation des Parameters oder der Parameter darin besteht, daß

(d1) bei vorgegebener Konzentration des O/W-Emulgators bzw. der Vielzahl an O/W-Emulgatoren sowie der Wasserphase und der Ölphase die Temperatur des Gemisches variiert wird, bzw. daß
(d2) bei vorgegebener Temperatur die Konzentration mindestens eines O/W-Emulgators, bzw. daß
(d3) bei vorgegebener Temperatur und vorgegebener Konzentration mindestens eines O/W-Emulgators, die Konzentration der Ölphase und oder die Konzentration der Wasserphase variiert wird.

Es kann erfindungsgemäß gegebenenfalls bevorzugt sein, mehrere Parameter gleichzeitig oder nacheinander zu variieren.

Die Herstellung der erfindungsgemäßen Mikroemulsionsgele erfolgt vorteilhaft auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe, welche unterhalb des Phaseninversionstemperaturbereiches eine O/W-Emulsion bilden, auf eine Temperatur bringt, welche mindestens der Schmelztemperatur der höchstschmelzenden Ölkomponente entspricht, aber gegebenenfalls unterhalb des Phaseninversionstemperaturbereiches des Systemes liegt, und die gebildete Mikroemulsion hernach auf Raumtemperatur abkühlt, wobei der oder die erfindungsgemäß verwendetenen Verdicker zu jedem Zeitpunkte der Herstellung beigefügt werden können. Der gesamte Vorgang vollzieht sich bevorzugt unter Rühren.

Die Herstellung der erfindungsgemäßen Mikroemulsionen erfolgt ebenfalls vorteilhaft dergestalt, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- undloder Wirkstoffe, welche unterhalb des Phaseninversionstemperaturbereiches eine O/W-Emulsion bilden, auf eine Temperatur oberhalb oder innerhalb des Phaseninversionstemperaturbereiches bringt, und die gebildete Mikroemulsion hernach auf Raumtemperatur abkühlt, wobei der oder die erfindungsgemäß verwendetenen Verdicker zu jedem Zeitpunkte der Herstellung beigefügt werden können. Der gesamte Vorgang vollzieht sich bevorzugt unter Rühren.

Erfindungsgemäß können vorteilhafte O/W-Mikroemulsionsgele erhalten werden, der Anteil des O/W-Emulgators unter 20 Gew.-%, insbesondere unter 15 Gew.-%, bezogen auf das Gesamtgewicht der Zubereitung liegt, weniger als 5 Gew.-% eines zusätzlichen W/O-Emulgators vorliegen, wobei der oder die erfindungsgemäß verwendeten Verdicker zu jedem Zeitpunkte der Herstellung beigefügt werden können.

Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Die erfindungsgemäßen Mikroemulsionsgele enthalten vorteilhaft Elektrolyte, insbesondere eines oder mehrere Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Die erfindungsgemäßen Mikroemulsionsgele tragen femer in vorzüglicher Weise zur Hautglättung bei, insbesondere, wenn sie mit einer oder mehreren Substanzen versehen sind, die die Hautglättung fördern.

Stellen die erfindungsgemäßen Mikroemulsionsgele Grundlagen für kosmetische Desodorantien/Antitranspirantien dar, so können alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, femer beispielsweise Zinksalze der Ricinolsäure. Keimhemmende Mittel sind ebenfalls geeignet, in die erfindungsgemäßen Mikroemulsionen eingearbeitet zu werden. Vorteilhafte Substanzen sind zum Beispiel 2,4,4'-Trichlor-2'hdroxydiphenylether (Irgasan), 1,6-Di-(4-chlorphenylbiguanido)-hexan (Chlorhexidin), 3,4,4'-Trichlorcarbanilid, quatemäre Ammoniumverbindungen, Nelkenöl, Minzöl, Thymianöl, Triethylcitrat, Famesol (3,7,11.Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Die üblichen Antitranspiranswirkstoffe können ebenfalls vorteilhaft in den erfindungsgemäßen Mikroemulsionsgele verwendet werden, insbesondere Adstringentien, beispielsweise basische Aluminiumchloride.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren Mikroemulsionsgelen.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Es hat sich darüberhinaus in überraschender Weise herausgestellt, daß bei der Verwendung von in der Ölphase löslichen Treibmitteln, also beispielsweise üblichen Propan-Butan-Gemischen, die erfindungsgemäßen O/W-Mikroemulsionsgele nicht einfach als Aerosoltröpfchen versprüht werden, sondern sich zu feinblasigen, reichhaltigen Schäumen entwickeln, sobald solche mit solchen Treibmitteln beladenen Systeme Druckentspannung erfahren.

Solche nachschäumenden Zubereitungen werden daher ebenfalls als vorteilhafte Verkörperungen der vorliegenden Erfindung mit eigenständiger erfinderischer Tätigkeit angesehen.

Bei der Verwendung von in der Ölphase unlöslichen Treibmitteln werden die erfindungsgemäßen O/W-Mikroemulsionsgele als Aerosoltröpfchen versprüht.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:

- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Satze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten. die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Mikroemulsionsgele mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe bestehend aus

Aminosäuren (z.B. Histidin, Tyrosin, Tryptophan) und deren Derivate, Imidazole (z.B. Urocaninsäure) und deren Derivate, Peptide wie D,L-Camosin, D-Camosin, L-Carnosin und deren Derivate (z.B. Anserin), Carotinoide, Carotine (z.B. $\alpha$-Carotin, $\beta$-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Satze) sowie Sulfoximinverbindungen (z.B. Buthioninsulfoximine, Homocysteinsulfoximin, Buthioninsulfone, Penta-, Hexa-, Heptahioninsulfoximin) in sehr geringen verträglichen Dosierungen (z.B. pmol bis $\mu$mol/kg), femer (Metall)-Chelatoren (z.B. $\alpha$-Hydroxyfettsäu-ren, $\alpha$-Hydroxypalmitinsäure, Phytinsäure, Lactoferrin), $\alpha$-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate. Mg - Ascorbylphosphate, Ascorbylacetate), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Zink und dessen Derivate (z.B. ZnO. $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0.001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die $\gamma$-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Obgleich selbstverständlich auch die Verwendung hydrophiler Wirkstoffe erfindungsgemäß begünstigt ist, ist ein weiterer Vorteil der erfindungsgemäßen Mikroemulsionsgele, daß die hohe Anzahl feinstzerteilter Tröpfchen gerade öllösliche bzw. lipophile Wirkstoffe mit besonders großer Wirksamkeit biologisch verfügbar macht.

Vorteilhaft ist es auch, die Wirkstoffe aus der Gruppe der rückfettenden Substanzen zu wählen, beispielsweise Purcellinöl, Eucerit® und Neocerit®.

Es ist auch möglich und gegebenenfalls vorteilhaft, den erfindungsgemäßen Zubereitungen waschaktive Tenside zuzufügen. Erfindungsgemäße wäßrige kosmetische Reinigungsmittel oder für die wäßrige Reinigung bestimmte wasserarme oder wasserfreie Reinigungsmittelkonzentrate können kationische, anionische, nichtionische und/oder amphotere Tenside enthalten, beispielsweiseherkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaïne, Sulfosuccinate, Sulfobemsteinsäurehalbester, Alkylethercarboxylate, Eiweiß-Fettsäure-Kondensate, Alkylbetaïne und Amidobetaine, Fettsäurealkanolamide, Polyglycolether-Derivate.

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, gegebenenfalls Elektrolyte und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen. Vorteilhaft sind beispielsweise Cetyltrimethylammoniumsalze zu verwenden.

Die erfindungsgemäßen für die Reinigung des Haares oder der Haut vorgesehenen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungsund Sequestrierungsagentien, Perlglanzagentien, Pflanzenextrakte, Vitamine, Wirkstoffe und dergleichen.

Die erfindungsgemäßen Zubereitungen haben, trotz ihres Ölgehaltes, in erstaunlicher Weise sehr gute Schaumentwicklung, hohe Reinigungskraft und wirken in hohem Maße regenerierend in bezug auf den allgemeinen Hautzustand. Insbesondere wirken die erfindungsgemäßen Zubereitungen hautglättend, vermindern das Trockenheitsgefühl der Haut und machen die Haut geschmeidig.

Sollen die erfindungsgemäßen Mikroemulsionsgele zur Haarpflege eingesetzt werden, können sie die üblichen Bestandteile enthalten, üblicherweise zum Beispiel filmbildende Polymere. Von solchen Polymeren mit wenigstens teilweise quatemisierten Stickstoffgruppen (im folgenden "Filmbildner" genannt), eigenen sich bevorzugt solche, welche gewählt werden aus der Gruppe der Substanzen, welche nach der INCI-Nomenklatur (International Nomenclature Cosmetic Ingredient) den Namen "Polyquaternium" tragen, beispielsweise:

| | |
|---|---|
| Polyquaternium-2 | (Chemical Abstracts-Nr. 63451-27-4, z.B. Mirapol® A-15) |
| Polyquaternium-5 | (Copolymeres aus dem Acrylamid und dem β-Methacryloxyethyltrimethylammoniummethosulfat, CAS-Nr. 26006-22-4) |
| Polyquaternium-6 | (Homopolymer des N,N-Dimethyl-N-2-propenyl-2-propen-1-aminiumchlorids, CAS-Nr. 26062-79-3, z.B. Merquat® 100 |
| Polyquaternium-7 | N,N-Dimethyl-N-2-propenyl-2-propen-1-aminiumchlorid, Polymeres mit 2-Propenamid, CAS-Nr. 26590-05-6, z.B. Merquat® S |
| Polyquaternium-10 | Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M, |
| Polyquaternium-11 | Vinylpyrrolidon/dimethylaminoethyl-Methacrylat-Copolymer/Diethylsulfat-Reaktionsprodukt, CAS-Nr. 53633-54-8, z.B. Gafquat® 755N |
| Polyquatemium-16 | Vinylpyrrolidon/vinylimidazoliniummethochlorid-Copolymer, CAS-Nr. 29297-55-0, z.B. Luviquat® HM 552 |
| Polyquatemium-17 | CAS-Nr. 90624-75-2, z.B. Mirapol® AD-1 |
| Polyquaternium-19 | Quaternisierter wasserlöslicher Polyvinylalkohol |
| Polyquatemium-20 | in Wasser dispergierbarer quatemisierter Polyvinyloctadecylether |
| Polyquatemium-21 | Polysiloxan-polydimethyl-dimethylammoniumacetat-Copolymeres, z.B. Abil® B 9905 |
| Polyquatemium-22 | Dimethyldiallylammoniumchlorid/Acrylsäure-Copolymer, CAS-Nr. 53694-7-0, z.B. Merquat® 280 |
| Polyquaternium-24 | Polymeres quatemäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200 |
| Polyquaternium-28 | Vinylpyrrolidon/Methacrylamidopropyltrimethylammoniumchlorid-Copolymer, z.B. Gafquat® HS-100 |
| Polyquaternium-29 | z.B. Lexquat® CH |
| Polyquatemium-31 | CAS-Nr. 136505-02-7, z.B. Hypan® QT 100 |
| Polyquaternium-32 | N,N,N-trimethyl-2-[(2-methyl-1-oxo-2-propenyl)oxy]-Ethanaminiumchlorid, polymer mit 2-Propenamid, CAS-Nr. 35429-19-7 |
| Polyquatemium-37 | CAS-Nr. 26161-33-1 |

Vorteilhaft enthalten erfindungsgemäße Zubereitungen zur Haarpflege 0.2 - 50 Gew.-% eines oder mehrerer Filmbildner, bevorzugt 5 - 30 Gew.-%, insbesondere 10 - 25 Gew.-%, jeweils bezogen auf das Gesamtgewicht der Zubereitungen. Derartige Ausführungsformen der erfindungsgemäßen Zubereitungen pflegen durch Umwelteinflüsse geschädigtes oder strapaziertes Haar bzw. beugen solchen Umwelteinflüssen vor. Ferner verleihen die erfindungsgemäßen Zubereitungen der Haartracht lockere Fülle und Festigkeit, ohne klebrig zu wirken.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, weitere, nicht unter die Definition der erfindungsgemäßen Verdicker fallende Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Insbesondere vorteilhaft werden Gemische der vorstehend genannten Lösungsmittel verwendet.

Als weitere Bestandteile können verwendet werden Fette, Wachse und andere natürliche und synthetische Fettkörper, vorzugsweise Ester von Fettsäuren mit Alkoholen niedriger C-Zaht. z.B. mit Isopropanol, Propylenglykol oder Glycerin, oder Ester von Fettalkoholen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether. Propylenglykolmonomethyl, -monoethyl- oder -monobutylether, Diethylenglykolmonomethyl- oder -monoethylether und analoge Produkte.

Die nachfolgenden Beispiele sollen die vorliegende Erfindung verdeutlichen.

**Beispiel 1**

| Desodorierende Zubereitung | Gew.-% |
|---|---|
| Glycerylisostearat | 2,400 |
| PEG-15-Cetylisostearylalkohol | 4,800 |
| Isotridecylisononanoat | 1,670 |
| PEG-300-Pentaerythrityltetraisostearat | 1,000 |
| Cyclomethicon | 3,300 |
| Butylenglycol | 3,000 |
| Famesol | 0,300 |
| Glycerinmonocaprat | 0,100 |
| Aluminiumchlorhydrat | 4,000 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 2**

| Desodorierende Zubereitung | Gew.-% |
|---|---|
| Glycerylisostearat | 1,800 |
| PEG-15-Cetylstearylalkohol | 5,200 |
| Sorbitol | 2,900 |
| Isotridecylisononanoat | 3,300 |

(fortgesetzt)

| Desodorierende Zubereitung | Gew.-% |
|---|---|
| PEG-300-Pentaerythrityltetraisostearat | 1,000 |
| Cyclomethicon | 6,600 |
| Glycerinmonocaprat | 0,100 |
| Aluminiumchlorhydrat | 3,883 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 3**

| Desodorierende Zubereitung | Gew.-% |
|---|---|
| Glycerylisostearat | 1,800 |
| PEG-17-Cetylstearylalkohol | 5,200 |
| PEG-300-Pentaerythrityltetraisostearat | 1,000 |
| Isotridecylisononanoat | 10,000 |
| Sorbitol | 2,900 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 4**

| Desodorierende Zubereitung | Gew.-% |
|---|---|
| Sorbitanmonoisostearat | 2,300 |
| PEG-15-Cetylstearylalkohol | 4,600 |
| Sorbitol | 2,900 |
| Cyclomethicon | 6,600 |
| PEG-300-Pentaerythrityltetraisostearat | 1,000 |
| Isotridecylisononanoat | 3,300 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 5**

| Desodorierende Zubereitung | Gew.-% |
|---|---|
| Digycerylmonoisostearat | 1,800 |
| PEG-15-Cetylstearylalkohol | 5,100 |
| $C_{12-15}$-Alkylbenzoat | 5,000 |
| PEG-300-Pentaerythrityltetraisostearat | 1,000 |
| Octylisostearat | 5,000 |
| Sorbitol | 2,900 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 6**

| Desodorierende Zubereitung | Gew.-% |
|---|---|
| Diglycerylmonoisostearat | 2,300 |
| PEG-15-Cetylstearylalkohol | 4,600 |
| Cyclomethicon | 6,600 |
| PEG-300-Pentaerythrityltetraisostearat | 1,000 |
| Sorbitol | 2,900 |
| Isotridecylisononanoat | 3,300 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 7**

| Desodorierende Zubereitung | Gew.-% |
|---|---|
| Glycerylisostearat | 1,800 |
| PEG-16-Stearylalkohol | 5,100 |
| Octylisostearat | 3,300 |
| PEG-300-Pentaerythrityltetraisostearat | 1,000 |
| Cyclomethicon | 6,600 |
| Sorbitol | 2,900 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 8**

| Desodorierende Zubereitung | Gew.-% |
|---|---|
| Propylenglycolmonoisostearat | 2,300 |
| PEG-15-Cetylstearylalkohol | 4,600 |
| Isotridecylisononanoat | 3,300 |
| PEG-300-Pentaerythrityltetraisostearat | 1,000 |
| Cyclomethicon | 6,600 |
| Sorbitol | 2,900 |
| Glycerinmonocaprat | 0,100 |
| Aluminiumchlorhydrat | 3,900 |
| Parfum, Antioxidantien | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 9**

| Lichtschutzzubereitung | Gew.-% |
|---|---|
| Glycerylisostearat | 2,400 |
| Isoceteth-20 | 4,800 |
| Cetearylisononanoat | 1,670 |
| PEG-300-Pentaerythrityltetraisostearat | 1,000 |
| Eusolex® 232 | 3,000 |
| Cyclomethicon | 3,330 |
| NaOH | 0,990 |
| Glycerin | 3,000 |
| Parfüm, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 10**

| Hautpflegegel | Gew.-% |
|---|---|
| Glycerylisostearat | 2,400 |
| PEG-60-Evening Primrose Glycerides | 4,800 |
| Cetylhydroxyethylcellulose | 4,000 |
| Isotridecylisononanoat | 3,340 |
| Cyclomethicon | 6,660 |
| Butylenglycol | 3,000 |
| Glycerinmonocaprinat | 0,100 |

(fortgesetzt)

| Hautpflegegel | Gew.-% |
|---|---|
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 11**

| Gesichtsreinigungsgel | Gew.-% |
|---|---|
| Glycerylisolaurat | 4.588 |
| Laureth-11-carboxylsäure (90 %) | 3,754 |
| Cetearylisononanoat | 1,773 |
| PEG-150-Distearat | 1,000 |
| Cyclomethicon | 3,441 |
| Butylenglycol | 3,128 |
| NaOH | 0,206 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 12**

| Körperpflegegel | Gew.-% |
|---|---|
| PEG-20-stearat | 4,800 |
| Glycerylisostearat | 2,400 |
| Isotridecylisononanoat | 6,660 |
| PEG-150-Distearat | 1,000 |
| Glycerinmonocaprinat | 0,100 |
| Cyclomethicon | 3,340 |
| Butylenglycol | 3,000 |
| Famesol | 0,300 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 13**

| Aftershave-Gel | Gew.-% |
|---|---|
| Sorbitanisostearat | 2,400 |
| Isotridecylisononanoat | 1,670 |

(fortgesetzt)

| Aftershave-Gel | Gew.-% |
|---|---|
| PEG-20-Sorbitanmonostearat | 4,800 |
| PEG-150-Distearat | 1,000 |
| Butylenglycol | 3,000 |
| Glycerinmonocaprinat | 0,100 |
| Cyclomethicon | 3,330 |
| Farnesol | 0,300 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 14**

| Reinigungsgel gegen fettige Haut | Gew.-% |
|---|---|
| Isotridecylisononanoat | 1,670 |
| PEG-20-Sorbitanmonooleat | 4,800 |
| Cyclomethicon | 3,330 |
| PEG-150-Distearat | 1,000 |
| Butylenglycol | 3,000 |
| Glycerinmonooleat | 2,400 |
| Famesol | 0,300 |
| Glycerinmonocaprinat | 0,100 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 15**

| Aftershave-Gel | Gew.-% |
|---|---|
| Isotridecylisononanoat | 3,311 |
| Glycerylisostearat | 1,786 |
| Oleth-15 | 5,146 |
| PEG-150-Distearat | 1,000 |
| Sorbitol | 2,913 |
| Glycerinmonocaprinat | 0,194 |
| Cyclomethicon | 6,621 |
| Famesol | 0,097 |
| Ethanol | 3,883 |
| Parfum. Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 16**

| Haargel | Gew.-% |
|---|---|
| Glycerylisostearat | 2,400 |
| Ceteareth-15 | 4,800 |
| PEG-150-Distearat | 1,000 |
| Caprylic/Capric/Triglycerides | 3,340 |
| Butylenglycol | 3,000 |
| Glycerinmonocaprinat | 0,100 |
| Cyclomethicon | 6,660 |
| Famesol | 0,300 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 17**

| Gesichtspflegegel | Gew.-% |
|---|---|
| Glycerylisostearat | 2,400 |
| PEG-15-Cetylstearylalkohol | 4,800 |
| PEG-150-Dioleat | 1,000 |
| Dicaprylylether | 5,000 |
| Butylenglycol | 3,000 |
| Glycerinmonocaprinat | 0,100 |
| Famesol | 0,300 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 18**

| Abschminkgel | Gew.-% |
|---|---|
| Diglycerinmonoisostearat | 1,840 |
| Ceteareth-15 | 5,300 |
| PEG-150-Dioleat | 1,000 |
| Paraffinum liquidum | 5,000 |
| Sorbitol | 3,000 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 19**

| Deo-Gel | Gew.-% |
|---|---|
| Ceteareth-15 | 5,146 |
| Octyldodecanol | 9,932 |
| PEG-150-Dioleat | 1,000 |
| Sorbitol | 2,913 |
| Farnesol | 0,097 |
| Diglycerinmonoisostearat | 1,786 |
| Glycerinmonocaprinat | 0,194 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 20**

| Gesichtspflegegel | Gew.-% |
|---|---|
| PEG-6 Caprylsäure/Caprinsäureglyceride | 4,800 |
| Isotridecylisononanoat | 1,670 |
| PEG-230-Glyceryltriisostearat | 1,000 |
| Butylenglycol | 3,000 |
| Glycerinmonocaprinat | 2,400 |
| Cyclomethicon | 3,330 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 21**

| | Gew.-% |
|---|---|
| Glycerylisostearat | 2,400 |
| Isotridecylisononanoat | 1,670 |
| Cyclomethicon | 3,330 |
| PEG-450-Sorbitolhexaisostearat | 1,000 |
| Butylenglycol | 3,000 |
| PEG-20-Glycerylisostearat | 4,800 |
| Famesol | 0,300 |
| Glycerinmonocaprinat | 0,100 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 22**

| Nachschäumendes Gel | Gew.-% |
|---|---|
| Glycerylisolaurat | 4,600 |
| Natrium-Laureth 1-4 Sulfat (25 %-ig) | 15,000 |
| PEG-150-Distearat | 2,000 |
| Cyclomethicon | 3,440 |
| Cetearylisononanoat | 1,770 |
| Butylenglycol | 3,125 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet. Die vorstehende Mischung wird mit dem Verdicker und der vierfachen Menge eines Gemisches Propan/Butan 2.7 versehen.

**Beispiel 23**

| Mikrodispersionsgel | Gew.-% |
|---|---|
| Glycerylisostearat | 2,400 |
| Ceteth-15 | 4,800 |
| PEG-150-Distearat | 1,000 |
| Cetylpalmitat | 4,000 |
| Butylenglycol | 3,000 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 24**

| Desodorierende Zubereitung | Gew.-% |
|---|---|
| Glycerylisostearat | 1,800 |
| Ceteareth-15 | 5,100 |
| PEG-120-Methylglucosedioleat | 1,000 |
| Cyclomethicon | 6,660 |
| Butylenglycol | 2,900 |
| Farnesol | 0,300 |
| Aluminiumchlorhydrat | 20,000 |
| Isotridecylisononanoat | 3,300 |
| Glycerinmonocaprinat | 0,200 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 25**

| Desodorierende Zubereitung | Gew.-% |
|---|---|
| Glycerylisostearat | 1,800 |
| Ceteareth-15 | 5,100 |
| PEG-160-Sorbitantriisostearat | 1,000 |
| Cyclomethicon | 6,660 |
| Sorbitol | 2,900 |
| Farnesol | 0,100 |
| Aluminiumchlorhydrat | 7,800 |
| Isotridecylisononanoat | 3,300 |
| Glycerinmonocaprinat | 0,200 |
| Parfum, Konservierungsmittel. Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Beispiel 26**

| Desodorierende Zubereitung | Gew.-% |
|---|---|
| Glycerylisostearat | 2,400 |
| Ceteareth-15 | 4,800 |
| Urethan $C_{1-20}$-Alkyl-PEG-Copolymer | 2,000 |
| Cyclomethicon | 6,660 |
| Butylenglycol | 3,000 |
| Famesol | 0,300 |
| Aluminiumchlorhydrat | 20,000 |
| Isotridecylisononanoat | 3,300 |
| Glycerinmonocaprinat | 0,100 |
| Parfum, Konservierungsmittel, Farbstoffe | q.s. |
| Wasser | ad 100,000 |

Die Ölphase und die Wasserphase werden getrennt auf je 85 - 90° C erhitzt, vereinigt und auf Raumtemperatur abgekühlt, wobei sich ein transparentes bis transluzentes O/W-Mikroemulsionsgel bildet.

**Patentansprüche**

**1.** Mikroemulsionsgele,

(a) beruhend auf Mikroemulsionen vom Typ Öl-in-Wasser, welche umfassen

- eine diskontiniuerliche Ölphase und eine kontinuierliche Wasserphase
- enthaltend mindestens einen polyethoxylierten bzw. polypropoxylierten bzw. polyethoxylierten und polypropoxylierten O/W-Emulgator
- wobei der polyethooxylierte bzw. polypropoxylierte bzw. polyethoxylierte und polypropoxylierte O/W-Emulgat oder die polyethooxylierten bzw. polypropoxylierten bzw. polyethoxylierten und polypropoxylierten O/W-Emulgatoren gewählt wird oder werden aus der Gruppe
- der Fettalkoholethoxylate der allgemeinen Formel R-O-(-CH$_2$-CH$_2$-O-)$_n$-H, wobei R einen verzweigten oder unverzweigten Alkyl-, Aryl- oder Alkenylrest und n eine Zahl von 10 bis 50 darstellen

- der ethoxylierten Wollwachsalkohole,

- der Polyethylenglycolether der allgemeinen Formel R-O-(-CH$_2$-CH$_2$-O-)$_n$-R', wobei R und R' unabhängig voneinander verzweigte oder unverzweigte Alkyl- oder Alkenylreste und n eine Zahl von 10 bis 80 darstellen

- der Fettsäureethoxylate der allgemeinen Formel R-COO-(-CH$_2$-CH$_2$-O-)$_n$-H, wobei R einen verzweigten oder unverzweigten Alkyl- oder Alkenylrest und n eine Zahl von 10 bis 40 darstellen,

- der veretherten Fettsäureethoxylate der allgemeinen Formel R-COO-(-CH$_2$-CH$_2$-O-)$_n$-R', wobei R und R' unabhängig voneinander verzweigte oder unverzweigte Alkyl- oder Alkenylreste und n eine Zahl von 10 bis 80 darstellen,

- der veresterten Fettsäureethoxylate der allgemeinen Formel R-COO-(-CH$_2$-CH$_2$-O-)$_n$-C(O)-R', wobei R und R' unabhängig voneinander verzweigte oder unverzweigte Alkyl- oder Alkenylreste und n eine Zahl von 10 bis 80 darstellen,

- der Polyethylenglycolglycerinfettsäureester gesättigter und/oder ungesättigter, verzweigter und/oder unverzweiger Fettsäuren und einem Ethoxylierungsgrad zwischen 3 und 50,

- der ethoxylierten Sorbitanester mit einem Ethoxylierungsgrad von 3 bis 100

- der Cholesterinethoxylate mit einem Ethoxylierungsgrad zwischen 3 und 50,

- der ethoxylierten Triglyceride mit einem Ethoxylierungsgrad zwischen 3 und 150,

- der Alkylethercarbonsäuren der allgemeinen Formel R-O-(-CH$_2$-CH$_2$-O-)$_n$-CH$_2$-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,

- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,

- der Alkylethersulfate bzw. die diesen Sulfaten zugrundeliegenden Säuren der allgemeinen Formel R-O-(-CH$_2$-CH$_2$-O-)$_n$-SO$_3$-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen.

- der Fettalkoholpropoxylate der allgemeinen Formel R-O-(-CH$_2$-CH(CH$_3$)-O-)$_n$-H, wobei R einen verzweigten oder unverzweigten Alkyl- oder Alkenylrest und n eine Zahl von 10 bis 80 darstellen,

- der Polypropylenglycolether der allgemeinen Formel R-O-(-CH$_2$-CH(CH$_3$)-O-)$_n$-R', wobei R und R' unabhängig voneinander verzweigte oder unverzweigte Alkyl- oder Alkenylreste und n eine Zahl von 10 bis 80 darstellen

- der propoxylierten Wollwachsalkohole,

- der veretherten Fettsäurepropoxylate der allgemeinen Formel R-COO-(-CH$_2$-CH(CH$_3$)-O-)$_n$-R', wobei R und R' unabhängig voneinander verzweigte oder unverzweigte Alkyl- oder Alkenylreste und n eine Zahl von 10 bis 80 darstellen,

- der veresterten Fettsäurepropoxylate der allgemeinen Formel R-COO-(-CH$_2$-CH(CH$_3$)-O-)$_n$-C(O)-R', wobei R und R' unabhängig voneinander verzweigte oder unverzweigte Alkyl- oder Alkenylreste und n eine Zahl von 10 bis 80 darstellen,

- der Fettsäurepropoxylate der allgemeinen Formel R-COO-(-CH$_2$-CH(CH$_3$)-O-)$_n$-H, wobei R einen verzweigten oder unverzweigten Alkyl- oder Alkenylrest und n eine Zahl von 10 bis 80 darstellen,

- der Polypropylenglycolglycerinfettsäureester gesättigter und/oder ungesättigter, verzweigter und/oder unverzweiger Fettsäuren und einem Propoxylierungsgrad zwischen 3 und 80

- der propoxylierten Sorbitanester mit einem Propoxylierungsgrad von 3 bis 100

- der Cholesterinpropoxylate mit einem Propoxylierungsgrad von 3 bis 100

- der propoxylierten Triglyceride mit einem Propoxylierungsgrad von 3 bis 100

- der Alkylethercarbonsäuren der allgemeinen Formel R-O-(-CH$_2$-CH(CH$_3$)O-)$_n$-CH$_2$-COOH bzw. deren kosmetisch oder pharmazeutisch akzeptablen Salze, wobei R einen verzweigten oder unverzweigten Alkyloder Alkenylrest und n eine Zahl von 3 bis 50 darstellen,

- der Alkylethersulfate bzw. die diesen Sulfaten zugrundeliegenden Säuren der allgemeinen Formel R-O-(-CH$_2$-CH(CH$_3$)-O-)$_n$-SO$_3$-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,

- der Fettalkoholethoxylate/propoxylate der allgemeinen Formel R-O-X$_n$-Y$_m$-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,

- der Polypropylenglycolether der allgemeinen Formel R-O-X$_n$-Y$_m$-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,

- der veretherten Fettsäurepropoxylate der allgemeinen Formel $R-COO-X_n-Y_m-R'$, wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,

- der Fettsäureethoxylate/propoxylate der allgemeinen Formel $R-COO-X_n-Y_m-H$, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

- gewünschtenfalls enthaltend einen oder mehrere W/O-Emulgatoren

- einen Emulgatorgehalt kleiner als 20 Gew.-%, bezogen auf das Gesamtgewicht der Emulsion, aufweisend,

- erhältlich auf die Weise, daß ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt,

(b) bei welcher die Tröpfchen der diskontinuierlichen Ölphase durch eine oder mehrere Vernetzersubstanzen miteinander verbunden sind, gewählt aus der Gruppe

- der Polyethylenglycolether der allgemeinen Formel $R-O-(-CH_2-CH_2-O-)_n-R'$, wobei R und R' unabhängig voneinander verzweigte oder unverzweigte Alkyl-, Aryl- oder Alkenylreste und n eine Zahl größer als 100 darstellen,

- der veretherten Fettsäureethoxylate der allgemeinen Formel $R-COO-(-CH_2-CH_2-O-)_n-R'$, wobei R und R' unabhängig voneinander verzweigte oder unverzweigte Alkyl-, Aryl- oder Alkenylreste und n eine Zahl größer als 100 darstellen,

- der veresterten Fettsäureethoxylate der allgemeinen Formel $R-COO-(-CH_2-CH_2-O-)_n-C(O)-R'$, wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel $R-O-(-CH_2-CH(CH_3)-O-)_n-R'$, wobei R und R' unabhängig voneinander verzweigte oder unverzweigte Alkyl-, Aryl- oder Alkenylreste und n eine Zahl größer als 100 darstellen, der veresterten Fettsäurepropoxylate der allgemeinen Formel $R-COO-(-CH_2-CH(CH_3)-O-)_n-C(O)-R'$, wobei R und R' unabhängig voneinander verzweigte oder unverzweigte Alkyl-, Aryl- oder Alkenylreste und n eine Zahl größer als 100 darstellen, der Polypropylenglycolether der allgemeinen Formel $R-O-X_n-Y_m-R'$, wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist

- der veretherten Fettsäurepropoxylate der allgemeinen Formel $R-COO-X_n-Y_m-R'$, wobei R und R' unabhängig voneinander verzweigte oder unverzweigte Alkyl-, Aryl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist.

2. Verfahren zur Herstellung von O/W-Mikroemulsionsgelen, gemäß Ansprüch 1 welche umfassen:

(1) eine Wasserphase, gewünschtenfalls umfassend übliche, in Wasser lösliche oder dispergierbare Substanzen,

(2) eine Ölphase, welche im wesentlichen aus schwerflüchtigen Bestandteilen zusammengesetzt ist und welche gewünschtenfalls übliche, in der Ölphase lösliche oder dispergierbare Substanzen umfaßt,

(3) einen oder mehrere polyethoxylierte O/W-Emulgatoren und/oder einen oder mehrere polypropoxylierte O/W-Emulgatoren und/oder einen oder mehrere polyethoxylierte und polypropoxylierte O/W-Emulgatoren,

(4) gewünschtenfalls einen oder mehrere W/O-Emulgatoren,

(5) eine oder mehrere Vernetzersubstanzen, deren Moleküle sich durch mindestens einen hydrophilen Bereich auszeichnen, welcher eine Ausdehnung aufweist, die geeignet ist, den Abstand zweier Mikroemulsionströpfchen untereinander zu überbrücken, und durch mindestens zwei hydrophobe Bereiche, welche mit Mikroemulsionströpfchen in hydrophobe Wechselwirkung zu treten vermögen,

wobei

(a) die Anfangskonzentrationen der Ölphase, der Wasserphase und gewünschtenfalls eines oder mehrerer W/O-Emulgatoren gewählt werden und diese Bestandteile zueinander gegeben werden,

(b) die Anfangskonzentration des oder der O/W-Emulgatoren, welche auch gegebenenfalls gleich Null sein kann, gewählt wird und dieser oder diese O/W-Emulgatoren zu dem in (a) erhaltenden Gemisch gegeben werden

(c) wobei das in (b) erhaltene Gemisch eine Ausgangstemperatur besitzt

(d) das in (b) erhaltende Gemisch durch geeignete Variation mindestens eines Parameters, gewählt aus der Gruppe Temperatur und der Konzentration bzw. Konzentrationen mindestens eines der gewählten Emulgatoren und/oder der Ölphase und/oder der Wasserphase,das so gebildete Gemisch den Phaseninversionsbereich zwischen W/O-Emulsionen und O/W-Emulsionen durchtäuft und in den Bereich gebracht wird, wo das Gemisch als O/W-Emulsion bzw. O/W-Mikroemulsion vorliegt,

(e) das in (d) erhaltene Gemisch sodann gegebenenfalls weiteren Aufbereitungsschritten unterworfen wird.

(f) wobei der oder die erfindungsgemäß verwendetenen Verdicker zu jedem Zeitpunkte der Herstellung beigefügt werden können.

**3.** Verfahren zur Herstellung von Mikroemulsionsgelen nach Anspruch 1, **dadurch gekennzeichnet, daß** ein Gemisch aus den Grundkomponenten, umfassend Wasserphase, Ölphase, einen oder mehrere der erfindungsgemäß verwendeten O/W-Emulgatoren, gewünschtenfalls einen oder mehrere W/O-Emulgatoren, einen oder mehrere deren Moleküle sich durch mindestens einen hydrophilen Bereich auszeichnen, welcher eine Ausdehnung aufweist, die geeignet ist, den Abstand zweier Mikroemulsionströpfchen untereinander zu überbrücken, und durch mindestens zwei hydrophobe Bereiche, welche mit Mikroemulsionströpfchen in hydrophobe Wechselwirkung zu treten vermögen, sowie gewünschtenfalls weitere Hilfs-, Zusatz- und/oder Wirkstoffe, welche unterhalb des Phaseninversionstemperaturbereiches eine O/W-Emulsion bilden, auf eine Temperatur bringt,

- bei welcher die in der Ölphase löslichen Komponenten entweder gelöst oder zurnindest in geschmolzenem Zustande vorliegen
- und welche mindestens der Schmelztemperatur der höchstschmelzenden, nicht in gelöstem Zustande vorliegenden Ölkomponente entspricht,
- welche unterhalb des Phaseninversionstemperaturbereiches des Systemes liegt,
- das entstandene Gemisch hernach auf Raumtemperatur abkühlt
- wobei die Vernetzersubstanz oder die Vernetzersubstanzen zu jedem Zeitpunkte der Herstellung beigefügt werden können.

### Claims

**1.** Microemulsion gels

(a) based on microemulsions of the oil-in-water type,
which comprise

- a discontinuous oily phase and a continuous aqueous phase
- comprising at least one polyethoxylated or polypropoxylated or polyethoxylated and polypropoxylated O/W emulsifier
- the polyethoxylated or polypropoxylated or polyethoxylated and polypropoxylated O/W emulsifier or the polyethoxylated or polypropoxylated or polyethoxylated and polypropoxylated O/W emulsifiers being chosen from the group consisting of
- the fatty alcohol ethoxylates of the general formula $R-O-(-CH_2-CH_2-O-)_n-H$, wherein R is a branched or unbranched alkyl, aryl or alkenyl radical and n is a number from 10 to 50,
- the ethoxylated wool wax alcohols
- the polyethylene glycol ethers of the general formula $R-O-(-CH_2-CH_2-O-)_n-R'$, wherein R and R' independently of one another are branched or unbranched alkyl or alkenyl radicals and n is a number from 10 to 80,
- the fatty acid ethoxylates of the general formula $R-COO-(-CH_2-CH_2-O-)_n-H$, wherein R is a branched or unbranched alkyl or alkenyl radical and n is a number from 10 to 40,
- the etherified fatty acid ethoxylates of the general formula $R-COO-(-CH_2-CH_2-O-)_n-R'$, wherein R and R' independently of one another are branched or unbranched alkyl or alkenyl radicals and n is a number from 10 to 80,

- the esterified fatty acid ethoxylates of the general formula R-COO-(-CH$_2$-CH$_2$-O-)$_n$-C(O)-R', wherein R and R' independently of one another are branched or unbranched alkyl or alkenyl radicals and n is a number from 10 to 80,

- the polyethylene glycol glycerol fatty acid esters of saturated and/or unsaturated, branched and/or unbranched fatty acids and [sic] a degree of ethoxylation of between 3 and 50,

- the ethoxylated sorbitan esters having a degree of ethoxylation of 3 to 100,

- the cholesterol ethoxylates having a degree of ethoxylation of between 3 and 50,

- the ethoxylated triglycerides having a degree of ethoxylation of between 3 and 150,

- the alkyl ether-carboxylic acids of the general formula R-O-(-CH$_2$-CH$_2$-O-)$_n$-CH$_2$-COOH or cosmetically or pharmaceutically acceptable salts thereof, wherein R is a branched or unbranched alkyl or alkenyl radical having 5 - 30 C atoms and n is a number from 5 to 30,

- the polyoxyethylene sorbitol fatty acid esters based on branched or unbranched alkanoic or alkenoic acids and having a degree of ethoxylation of 5 to 100, for example of the sorbeth type,

- the alkyl ether-sulphates and the acids on which these sulphates are based, of the general formula R-O-(-CH$_2$-CH$_2$-O-)$_n$-SO$_3$-H, with cosmetically or pharmaceutically acceptable cations, wherein R is a branched or unbranched alkyl or alkenyl radical having 5 - 30 C atoms and n is a number from 1 to 50,

- the fatty alcohol propoxylates of the general formula R-O-(-CH$_2$-CH(CH$_3$)-O-)$_n$-H, wherein R is a branched or unbranched alkyl or alkenyl radical and n is a number from 10 to 80,

- the polypropylene glycol ethers of the general formula R-O-(-CH$_2$-CH(CH$_3$)-O-)$_n$-R', wherein R and R' independently of one another are branched or unbranched alkyl or alkenyl radicals and n is a number from 10 to 80,

- the propoxylated wool wax alcohols,

- the etherified fatty acid propoxylates of the general formula R-COO-(-CH$_2$-CH(CH$_3$)-O-)$_n$-C(O)-R', wherein R and R' independently of one another are branched or unbranched alkyl or alkenyl radicals and n is a number from 10 to 80,

- the esterified fatty acid propoxylates of the general formula R-COO-(-CH$_2$-CH(CH$_3$)-O-)$_n$-H, wherein R and R' independently of one another are branched or unbranched alkyl or alkenyl radicals and n is a number from 10 to 80,

- the fatty acid propoxylates of the general formula R-COO-(-CH$_2$-CH(CH$_3$)-O-)$_n$-H, wherein R is a branched or unbranched alkyl or alkenyl radical and n is a number from 10 to 80,

- the polypropylene, glycol glycerol fatty acid esters of saturated and/or unsaturated, branched and/or unbranched fatty acids and [sic] a degree of propoxylation of between 3 and 80,

- the propoxylated sorbitan esters having a degree of propoxylation of 3 to 100,

- the cholesterol propoxylates having a degree of propoxylation of 3 to 100,

- the propoxylated triglycerides having a degree of propoxylation of 3 to 100,

- the alkyl ether-carboxylic acids of the general formula R-O-(-CH$_2$-CH(CH$_3$)-O-)$_n$-CH$_2$-COOH and cosmetically or pharmaceutically acceptable salts thereof, wherein R is a branched or unbranched alkyl or alkenyl radical and n is a number from 3 to 50,

- the alkyl ether-sulphates and the acids on which these sulphates are based, of the general formula R-O-(-CH$_2$-CH(CH$_3$)-O-)$_n$-SO$_3$-H, with cosmetically or pharmaceutically acceptable cations, wherein R is a branched or unbranched alkyl or alkenyl radical having 5 - 30 C atoms and n is a number from 1 to 50,

- the fatty alcohol ethoxylates/propoxylates of the general formula R-O-X$_n$-Y$_m$-H, wherein R is a branched or unbranched alkyl or alkenyl radical, and wherein X and Y are not identical and are in each case either an oxyethylene group or an oxypropylene group and n and m independently of one another are numbers from 5 to 50,

- the polypropylene glycol ethers of the general formula R-O-X$_n$-Y$_m$-R', wherein R and R' independently of one another are branched or unbranched alkyl or alkenyl radicals, and wherein X and Y are not identical and are in each case either an oxyethylene group or an oxypropylene group and n and m independently of one another are numbers from 5 to 100,

- the etherified fatty acid propoxylates of the general formula R-COO-X$_n$-Y$_m$-R', wherein R and R' independently of one another are branched or unbranched alkyl or alkenyl radicals, and wherein X and Y are not identical and are in each case either an oxyethylene group or an oxypropylene group and n and m independently of one another are numbers from 5 to 100,

- the fatty acid ethoxylates/propoxylates of the general formula R-COO-X$_n$-Y$_m$-H, wherein R is a branched or unbranched alkyl or alkenyl radical and wherein X and Y are not identical and are in each case either an oxyethylene group or an oxypropylene group and n and m independently of one another are numbers from 5 to 50,

- if desired comprising one or more W/O emulsifiers,
- having an emulsifier content of less than 20% by weight, based on the total weight of the emulsion,
- are obtainable by bringing a mixture of the base components, comprising the aqueous phase, the oily phase, one or more of the O/W emulsifiers according to the invention, if desired, one or more W/O emulsifiers, and if desired further auxiliaries, additives and/or active compounds, to a temperature within or above the phase inversion temperature range, and thereafter cooling it to room temperature,

(b) those in which the droplets of the discontinuous oily phase are joined to one another by one or more crosslinking substances chosen from the group consisting of

- the polyethylene glycol ethers of the general formula R-O-(-$CH_2$-$CH_2$-O-)$_n$-R', wherein R and R' independently of one another are branched or unbranched alkyl, aryl or alkenyl radicals and n is a number greater than 100,
- the etherified fatty acid ethoxylates of the general formula R-COO-(-$CH_2$-$CH_2$-O-)$_n$-R', wherein R and R' independently of one another are branched or unbranched alkyl, aryl or alkenyl radicals and n is a number greater than 100,
- the esterified fatty acid ethoxylates of the general formula R-COO-(-$CH_2$-$CH_2$-O-)$_n$-C(O)-R', wherein R and R' independently of one another are branched or unbranched alkyl, aryl or alkenyl radicals and n is a number greater than 100,
- the polypropylene glycol ethers of the general formula R-O-(-$CH_2$-$CH(CH_3)$-O-)$_n$-R', wherein R and R' independently of one another are branched or unbranched alkyl, aryl or alkenyl radicals and n is a number greater than 100,
- the esterified fatty acid propoxylates of the general formula R-COO-(-$CH_2$-$CH(CH_3)$-O-)$_n$-C(O)-R', wherein R and R' independently of one another are branched or unbranched alkyl, aryl or alkenyl radicals and n is a number greater than 100,
- the polypropylene glycol ethers of the general formula R-O-$X_n$-$Y_m$-R', wherein R and R' independently of one another are branched or unbranched alkyl, aryl or alkenyl radicals, and wherein X and Y are not identical and are in each case either an oxyethylene group or an oxypropylene group and n and m independently of one another are numbers, the sum of which is greater than 100 [lacuna]
- the etherified fatty acid propoxylates of the general formula R-COO-$X_n$-$Y_m$-R', wherein R and R' independently of one another are branched or unbranched alkyl, aryl or alkenyl radicals, and wherein X and Y are not identical and are in each case either an oxyethylene group or an oxypropylene group and n and m independently of one another are numbers, the sum of which is greater than 100.

2. Process for the preparation of O/W microemulsion gels according to Claim 1, which comprise:

(1) an aqueous phase, if desired comprising customary substances which are soluble or dispersible in water,
(2) an oily phase which is composed essentially of constituents of low volatility and which, if desired, comprises customary substances which are soluble or dispersible in the oily phase,
(3) one or more polyethoxylated O/W emulsifiers and/or one or more polypropoxylated O/W emulsifiers and/or one or more polyethoxylated and polypropoxylated O/W emulsifiers,
(4) if desired, one or more W/O emulsifiers,
(5) one or more crosslinking substances, the molecules of which are distinguished by at least one hydrophilic region, which has an extension which is capable of bridging the distance between two microemulsion droplets, and by at least two hydrophobic regions, which are capable of entering into a hydrophobic interaction with the microemulsion droplets

where

(a) the initial concentrations of the oily phase, the aqueous phase and, if desired, one or more W/O emulsifiers are chosen and these constituents are added to one another,
(b) the initial concentration of the O/W emulsifier or emulsifiers, which can also be zero, if appropriate, is chosen and this or these O/W emulsifier or emulsifiers are added to the mixture obtained in (a),
(c) the mixture obtained in (b) having a starting temperature,
(d) the mixture obtained in (b), by suitable variation of at least one parameter chosen from the group consisting of temperature and the concentration or concentrations of at least one of the emulsifiers chosen and/or of the oily phase and/or of the aqueous phase, passes through the mixture thus formed [sic] the phase inversion range between W/O emulsions and O/W emulsions and is brought into the range where the mixture is present as an O/W emulsion or O/W microemulsion,

(e) the mixture obtained in (d) is then subjected to further processing steps, if appropriate,

(f) it being possible for the thickener or thickeners used according to the invention to be added at any point in time of the preparation.

**3.** Process for the preparation of microemulsion gels according to Claim 1, **characterized in that** a mixture of the base components, comprising the aqueous phase, the oily phase, one or more of the O/W emulsifiers used according to the invention, if desired one or more W/O emulsifiers, one or more [lacuna], the molecules of which are distinguished by at least one hydrophilic region, which has an extension which is capable of bridging the distance between two microemulsion droplets, and by at least two hydrophobic regions, which are capable of entering into a hydrophobic interaction with the microemulsion droplets and if desired further auxiliaries, additives and/or active compounds which form an O/W emulsion below the phase inversion temperature range, is brought to a temperature

- at which the components which are soluble in the oily phase are present either in the dissolved form or at least in the molten state
- and which corresponds to at least the melting point of the highest-melting oil component which is not present in the dissolved state, and
- which is below the phase inversion temperature range of the system and
- the resulting mixture is then cooled to room temperature
- it being possible for the crosslinking substance or the crosslinking substances to be added at any point in time of the preparation.

**Revendications**

**1.** Gels de microémulsions

(a) à base de microémulsions du type huile-dans-eau, qui comprennent

- une phase huileuse discontinue et une phase aqueuse continue
- contenant au moins un émulsifiant H/E polyéthoxylé ou polypropoxylé ou polyéthoxylé et polypropoxylé,
- l'émulsifiant H/E polyéthoxylé ou polypropoxylé ou polyéthoxylé et polypropoxylé ou les émulsifiants H/E polyéthoxylés ou polypropoxylés ou polyéthoxylés et polypropoxylés étant choisi(s) dans le groupe,
- des produits d'éthoxylation d'alcools gras de formule générale, $R\text{-}O\text{-}(\text{-}CH_2\text{-}CH_2\text{-}O\text{-})_n\text{-}H$, dans laquelle R représente un radical alkyle, aryle ou alcényle ramifié ou non ramifié et n représente un nombre allant de 10 à 50,
- des alcolanums éthoxylés,
- des polyéthylèneglycoléthers de formule générale $R\text{-}O\text{-}(\text{-}CH_2\text{-}CH_2\text{-}O\text{-})_n\text{-}R'$, dans laquelle R et R' représentent, indépendamment l'un de l'autre, des radicaux alkyle ou alcényle ramifiés ou non ramifiés, et n représente un nombre allant de 10 à 80,
- des produits d'éthoxylation d'acides gras de formule générale $R\text{-}COO\text{-}(\text{-}CH_2\text{-}CH_2\text{-}O\text{-})_n\text{-}H$, dans laquelle R représente un radical alkyle ou alcényle ramifié ou non ramifié, et n représente un nombre allant de 10 à 40,
- des produits d'éthoxylation d'acides gras éthérifiés de formule générale $R\text{-}COO\text{-}(\text{-}CH_2\text{-}CH_2\text{-}O\text{-})_n\text{-}R'$, dans laquelle R et R' représentent, indépendamment l'un de l'autre, des radicaux alkyle ou alcényle ramifiés ou non ramifiés, et n représente un nombre allant de 10 à 80,
- des produits d'éthoxylation d'acides gras estérifiés de formule générale $R\text{-}COO\text{-}(\text{-}CH_2\text{-}CH_2\text{-}O\text{-})_n\text{-}C(O)\text{-}R'$, dans laquelle R et R' représentent, indépendamment l'un de l'autre, des radicaux alkyle ou alcényle ramifiés ou non ramifiés, et n représente un nombre allant de 10 à 80,
- des polyéthylèneglycol-esters d'acides gras et de glycérol, d'acides gras ramifiés et/ou non ramifiés, saturés et/ou insaturés, ayant un degré d'éthoxylation compris entre 3 et 50,
- des esters de sorbitanne éthoxylés ayant un degré d'éthoxylation allant de 3 à 100,
- des produits d'éthoxylation de cholestérol ayant un degré d'éthoxylation compris entre 3 et 50,
- des triglycérides éthoxylés ayant un degré d'éthoxylation compris entre 3 et 150,
- des acides alkyléthercarboxyliques de formule générale $R\text{-}O\text{-}(\text{-}CH_2\text{-}CH_2\text{-}O\text{-})_n\text{-}CH_2\text{-}COOH$, ou de leurs sels cosmétiquement ou pharmaceutiquement acceptables, R représentant un radical alkyle ou alcényle ramifié ou non ramifié ayant de 5 à 30 atomes de carbone, et n représentant un nombre allant de 5 à 30,
- des esters polyoxyéthyléniques d'acides gras de sorbitol, à base d'acides alcanoïques ou alcénoïques ramifiés ou non ramifiés et ayant un degré d'éthoxylation de 5 à 100, par exemple du type sorbeth,

- des alkyléthersulfates ou des acides à la base de ces sulfates, de formule générale R-O-(-CH$_2$-CH$_2$-O-)$_n$-SO$_3$-H, avec des cations cosmétiquement ou pharmaceutiquement acceptables, R représentant un radical alkyle ou alcényle ramifié ou non ramifié ayant de 5 à 30 atomes de carbone, et n représentant un nombre allant de 1 à 50,
- des produits de propoxylation d'alcools gras de formule générale R-O-(-CH$_2$-CH(CH$_3$)-O-)$_n$-H, dans laquelle R représente un radical alkyle ou alcényle ramifié ou non ramifié, et n représente un nombre allant de 10 à 80,
- des polypropylèneglycoléthers, de formule générale R-O-(-CH$_2$-CH(CH$_3$)-O-)$_n$-R', dans laquelle R et R' représentent, indépendamment l'un de l'autre, des radicaux alkyle ou alcényle ramifiés ou non ramifiés, et n représente un nombre allant de 10 à 80,
- des alcolanums propoxylés,
- des produits de propoxylation d'acides gras éthérifiés, de formule générale R-COO-(-CH$_2$-CH(CH$_3$)-O-)$_n$-R', dans laquelle R et R' représentent, indépendamment l'un de l'autre, des radicaux alkyle ou alcényle ramifiés ou non ramifiés, et n représente un nombre allant de 10 à 80,
- des produits de propoxylation d'acides gras estérifiés, de formule générale R-COO-(-CH$_2$-CH(CH$_3$)-O-)$_n$-C(O)-R', dans laquelle R et R' représentent, indépendamment l'un de l'autre, des radicaux alkyle ou alcényle ramifiés ou non ramifiés, et n représente un nombre allant de 10 à 80,
- des produits de propoxylation d'acides gras de formule générale R-COO-(-CH$_2$-CH(CH$_3$)-O-)$_n$-H, dans laquelle R représente un radical alkyle ou alcényle ramifié ou non ramifié, et n représente un nombre allant de 10 à 80,
- des polypropylèneglycol-esters d'acides gras et de glycérol, d'acides gras saturés et/ou insaturés, ramifiés et/ou non ramifiés et ayant un degré de propoxylation compris entre 3 et 80,
- des esters propoxylés de sorbitanne ayant un degré de propoxylation de 3 à 100,
- des produits de propoxylation de cholestérol ayant un degré de propoxylation de 3 à 100,
- des triglycérides propoxylés ayant un degré de propoxylation de 3 à 100,
- des acides alkyléthercarboxyliques de formule générale R-O-(-CH$_2$-CH(CH$_3$)O-)$_n$-CH$_2$-COOH ou de leurs sels cosmétiquement ou pharmaceutiquement acceptables, R représentant un radical alkyle ou alcényle ramifié ou non ramifié, et n représentant un nombre allant de 3 à 50,
- des alkyléthersulfates ou des acides à la base de ces sulfates, de formule générale R-O-(-CH$_2$-CH(CH$_3$)-O-)$_n$-SO$_3$-H, avec des cations cosmétiquement ou pharmaceutiquement acceptables, R représentant un radical alkyle ou alcényle ramifié ou non ramifié ayant de 5 à 30 atomes de carbone, et n représentant un nombre allant de 1 à 50,
- des produits d'éthoxylation/propoxylation d'alcools gras, de formule générale R-O-X$_n$-Y$_m$-H, R représentant un radical alkyle ou alcényle ramifié ou non ramifié, X et Y n'étant pas identiques et représentant chacun soit un groupe oxyéthylène, soit un groupe oxypropylène, et n et m représentant, indépendamment l'un de l'autre, des nombres allant de 5 à 50,
- des polypropylèneglycoléthers de formule générale R-O-X$_n$-Y$_m$-R', dans laquelle R et R' représentent, indépendamment l'un de l'autre, des radicaux alkyle ou alcényle ramifiés ou non ramifiés, X et Y n'étant pas identiques et représentant chacun soit un groupe oxyéthylène, soit un groupe oxypropylène, et n et m représentent, indépendamment l'un de l'autre, des nombres allant de 5 à 100,
- des produits de propoxylation d'acides gras éthérifiés de formule générale R-COO-X$_n$-Y$_m$-R', dans laquelle R et R' représentent, indépendamment l'un de l'autre, des radicaux alkyle ou alcényle ramifiés ou non ramifiés, X et Y ne sont pas identiques et représentent chacun soit un groupe oxyéthylène, soit un groupe oxypropylène, et n et m représentent, indépendamment l'un de l'autre, des nombres allant de 5 à 100,
- des produits d'éthoxylation/propoxylation d'acides gras, de formule générale R-COO-X$_n$-Y$_m$-H, dans laquelle R représente un radical alkyle ou alcényle ramifié ou non ramifié, X et Y ne sont pas identiques et représentent chacun soit un groupe oxyéthylène, soit un groupe oxypropylène, et n et m représentent, indépendamment l'un de l'autre, des nombres allant de 5 à 50,
- si on le désire, contenant un ou plusieurs émulsifiants E/H
- présentant une teneur en émulsifiants inférieure à 20 % en poids, par rapport au poids total de l'émulsion,
- pouvant être obtenus en portant à une température dans ou au-dessus de la plage de température d'inversion de phases un mélange des composants de base, comprenant phase aqueuse, phase huileuse, un ou plusieurs des émulsifiants H/E selon l'invention, si on le désire un ou plusieurs émulsifiants E/H, ainsi que, si on le désire, d'autres adjuvants, additifs et/ou substances actives, et ensuite en refroidissant jusqu'à la température ambiante,

(b) dans lesquels les gouttelettes de la phase huileuse discontinue sont reliées les unes aux autres par une ou plusieurs substances de réticulation choisies dans le groupe

- des polyéthylèneglycoléthers de formule générale R-O-(-CH$_2$-CH$_2$-O-)$_n$-R', dans laquelle R et R' représentent, indépendamment l'un de l'autre, des radicaux alkyle, aryle ou alcényle ramifiés ou non ramifiés, et n représente un nombre supérieur à 100,
- des produits d'éthoxylation d'acides gras éthérifiés de formule générale R-COO-(-CH$_2$-CH$_2$-O-)$_n$-R', dans laquelle R et R' représentent, indépendamment l'un de l'autre, des radicaux alkyle, aryle ou alcényle ramifiés ou non ramifiés, et n représente un nombre supérieur à 100,
- des produits d'éthoxylation d'acides gras estérifiés de formule générale R-COO-(-CH$_2$-CH$_2$-O-)$_n$-C(O)-R', dans laquelle R et R' représentent, indépendamment l'un de l'autre, des radicaux alkyle, aryle ou alcényle ramifiés ou non ramifiés, et n représente un nombre supérieur à 100, des polypropylèneglycoléthers de formule générale R-O-(-CH$_2$-CH(CH$_3$)-O-)$_n$-R', dans laquelle R et R' représentent, indépendamment l'un de l'autre, des radicaux alkyle, aryle ou alcényle ramifiés ou non ramifiés, et n représente un nombre supérieur à 100, des produits de propoxylation d'acides gras estérifiés de formule générale R-COO-(-CH$_2$-CH(CH$_3$)-O-)$_n$-C(O)-R', dans laquelle R et R' représentent, indépendamment l'un de l'autre, des radicaux alkyle, aryle ou alcényle ramifiés ou non ramifiés, et n représente un nombre supérieur à 100, des polypropylèneglycoléthers de formule générale R-O-X$_n$-Y$_m$-R', dans laquelle R et R' représentent, indépendamment l'un de l'autre, des radicaux alkyle, aryle ou alcényle ramifiés ou non ramifiés, X et Y ne sont pas identiques et représentent chacun soit un groupe oxyéthylène, soit un groupe oxypropylène, et n et m représentent, indépendamment l'un de l'autre, des nombres dont la somme est supérieure à 100,
- des produits de propoxylation d'acides gras éthérifiés de formule générale R-COO-X$_n$-Y$_m$-R', dans laquelle R et R' représentent, indépendamment l'un de l'autre, des radicaux alkyle, aryle ou alcényle ramifiés ou non ramifiés, X et Y ne sont pas identiques et représentent chacun soit un groupe oxyéthylène, soit un groupe oxypropylène, et n et m représentent, indépendamment l'un de l'autre, des nombres dont la somme est supérieure à 100.

2. Procédé pour la préparation de gels de microémulsions H/E selon la revendication 1, qui comprennent

(1) une phase aqueuse, si on le désire comprenant des substances usuelles, solubles ou dispersables dans l'eau,

(2) une phase huileuse qui est essentiellement composée de composants peu volatils et qui comprend si on le désire des substances usuelles, solubles ou dispersables dans la phase huileuse,

(3) un ou plusieurs émulsifiants H/E polyéthoxylés et/ou

un ou plusieurs émulsifiants H/E polypropoxylés et/ou

un ou plusieurs émulsifiants H/E polyéthoxylés et polypropoxylés,

(4) si on le désire un ou plusieurs émulsifiants H/E,

(5) une ou plusieurs substances de réticulation dont les molécules se distinguent par au moins une zone hydrophile qui présente une extension qui est appropriée à relier par un pont la distance entre deux gouttelettes de microémulsion, et par au moins deux zones hydrophobes qui peuvent entrer en interaction hydrophobe avec de gouttelettes de microémulsion,

dans lequel

(a) on choisit les concentrations initiales de la phase huileuse, de la phase aqueuse et, si on le désire, d'un ou plusieurs émulsifiants E/H, et on ajoute successivement ces composants,

(b) on choisit la concentration initiale de l'émulsifiant H/E ou des émulsifiants H/E, qui peut également être éventuellement égale à zéro, et on ajoute cet émulsifiant H/E ou ces émulsifiants H/E au mélange obtenu en (a),

(c) ce par quoi le mélange obtenu en (b) présente une température initiale,

(d) le mélange obtenu en (b), par une variation appropriée d'au moins un paramètre choisi dans le groupe constitué par la température et la concentration ou les concentrations d'au moins un des émulsifiants choisis et/ou de la phase huileuse et/ou de la phase aqueuse, le mélange ainsi formé traverse la zone d'inversion de phases entre émulsions E/H et émulsions H/E, et est amené dans la zone où le mélange se trouve sous forme d'émulsion H/E ou de microémulsion H/E,

(e) le mélange obtenu en (d) est ensuite éventuellement soumis à d'autres étapes de traitement final,

(f) l'épaississant ou les épaississants utilisés selon l'invention pouvant être ajoutés à tout instant de la préparation.

3. Procédé pour la préparation de gels de microémulsions selon la revendication 1, **caractérisé en ce qu'**on porte un mélange des composants de base comprenant phase aqueuse, phase huileuse, un ou plusieurs des émulsifiants H/E utilisés selon l'invention, si on le désire un ou plusieurs émulsifiants E/H, dont les molécules se distinguent par au moins une zone hydrophile qui présente une extension qui est appropriée à relier par un pont la distance entre des particules de microémulsions, et par au moins deux zones hydrophobes qui peuvent entrer en interaction hydrophobe avec des gouttelettes de microémulsions, ainsi que, si on le désire, d'autres adjuvants, additifs et/ou substances actives qui forment au-dessous de la zone d'inversion de phases une émulsion H/E, à une température

- à laquelle les composants solubles dans la phase huileuse se trouvent soit dissous, soit au moins à l'état fondu
- et qui correspond à au moins la température de fusion du composant huileux à plus haut point de fusion, ne se trouvant pas à l'état dissous,
- qui se trouve au-dessous la zone de température d'inversion de phases du système,
- le mélange résultant est ensuite refroidi jusqu'à la température ambiante,
- la substance de réticulation ou les substances de réticulation pouvant être ajoutées à tout instant de la préparation.

Fig. 1

W/O-Emulsionen

$\Theta$

$\Phi$

O/W-Emulsionen

P

EP 0 814 753 B1

Fig. 2

Fig. 3

Fig. 4

Fig. 5